# EUROPEAN PATENT SPECIFICATION

(11) **EP 2 859 121 B1**
(45) Date of publication and mention of the grant of the patent: **30.11.2016**
(21) Application number: 13801106.9
(22) Date of filing: 07.06.2013
(51) Int. Cl.: C12Q 1/68, C07H 21/00, G01N 33/543

(54) **APTAMER-BASED MULTIPLEXED ASSAYS**
APTAMERBASIERTE MULTIPLEXTESTS
ESSAIS MULTIPLEXÉS À BASE D'APTAMÈRES

(30) Priority: 07.06.2012 US 201261656956 P
(43) Date of publication of application: 15.04.2015
(73) Proprietor: Somalogic, Inc., Boulder, CO 80301 (US)
(72) Inventor: SANDERS, Glenn, Boulder, CO 80305 (US); KRAEMER, Stephan, Boulder, CO 80305 (US); KATILIUS, Evaldas, Superior, CO 80027 (US)
(74) Representative: Clegg, Richard Ian
(86) International application number: PCT/US2013/044792
(87) International publication number: WO 2013/185078

(56) References cited:
- WO-A2-2004/009798
- US-A1- 2003 224 366
- US-A1- 2009 042 206
- US-B1- 6 544 776
- LARRY GOLD ET AL: "Aptamer-Based Multiplexed Proteomic Technology for Biomarker Discovery", PLOS ONE, vol. 5, no. 12, 7 December 2010 (2010-12-07), page e15004, XP055040606, DOI: 10.1371/journal.pone.0015004
- GOLD, L ET AL.: 'Aptamer-Based Multiplexed Proteomic Technology For Biomarker Discovery.' PLOS ONE. vol. 5, 07 December 2010, pages 1 - 17, XP055040606

## Description

### FIELD OF THE INVENTION

The present invention relates generally to methods, devices, reagents and kits designed to improve the performance of multiplexed aptamer-based assays. Such methods have a wide utility in diagnostic applications as well as in biomarker discovery and the design and development of tools for research and development and aptamer-based therapeutics. Specifically, materials and methods are provided for the reduction or elimination of background signal.

### BACKGROUND

The following description provides a summary of information relevant to the present disclosure and is not a concession that any of the information provided or publications referenced herein is prior art to the presently claimed invention.

Assays directed to the detection and quantification of physiologically significant molecules in biological samples and other samples are important tools in scientific research and in the health care field. One class of such assays involves the use of a microarray that includes one or more aptamers immobilized on a solid support. The aptamers are each capable of binding to a target molecule in a highly specific manner and with very high affinity. See, *e.g.,* U.S. Patent No. 5,475,096 entitled "Nucleic Acid Ligands," see also, *e.g.,* U.S. Patent No. 6,242,246, U.S. Patent No. 6,458,543 and U.S. Patent No. 6,503,715, each of which is entitled "Nucleic Acid Ligand Diagnostic Biochip". Once the microarray is contacted with a sample, the aptamers bind to their respective target molecules present in the sample and thereby enable a determination of the absence, presence, amount, and/or concentration of the target molecules in the sample.

Multiplexed aptamer assays that provide solution-based target interaction and separation steps designed to remove specific components of an assay mixture have also been described, see U.S. Patent Nos. 7,855,054 and 7,964,356 and U.S. Publication Nos. US/2011/0136099 and US/2012/0115752. The aptamer assay methods described use one or more specific capture steps to separate components of a test sample from the target or targets to be detected while isolating the aptamer-target affinity complex.

The sensitivity and specificity of many assay formats are limited by the ability of the detection method to resolve true signal from signal that arises due to non-specific associations during the assay and result in a detectable signal. This is particularly true for multiplexed assays based on aptamers. It has been observed that one of the main sources of non-specific binding in this type of assay is a function of unanticipated aptamer-aptamer interactions. As target/aptamer interaction is dependent on maintaining the structural features of the target specific aptamer, any method to reduce aptamer-aptamer interactions needs to be balanced so as not to reduce the specific/target aptamer interactions. This disclosure describes methods to eliminate background in a single or multiplexed aptamer assay while maintaining target/aptamer specific interactions.

### SUMMARY

The present disclosure provides methods, devices, reagents, and kits designed to improve the performance of single analyte and multiplexed aptamer-based assays. Specifically, materials and methods are provided for the reduction or elimination of background signal.

In one embodiment, aptamers are provided that have high affinity and specificity for a target molecule and a first releasable tag. In some embodiments, the aptamers are photoaptamers. In some embodiments, this first releasable tag is a photocleavable biotin. Other tags and cleavable moieties and aptamer containing such tags and cleavable moieties are described.

The aptamer comprising the first releasable first tag that has a specific affinity for a target molecule is immobilized on a solid support in solution prior to equilibration binding with the test sample. The attachment of the aptamer to the solid support is accomplished by contacting a first solid support with the aptamer and allowing the releasable first tag included on the aptamer to associate, either directly or indirectly, with an appropriate first capture agent that is attached to or part of the first solid support. After attachment, washes with a solution buffered to pH 11 remove aptamer/aptamer aggregates, thereby reducing assay background ("Catch-0" immobilization, definition below)

A test sample is then prepared and contacted with the immobilized aptamers that have a specific affinity for their respective target molecules. If the test sample contains the target molecule(s), an aptamer-target affinity complex will form in the mixture with the test sample. Note that in addition to aptamer-target affinity complexes, uncomplexed aptamer will also be attached to the first solid support. The aptamer-target affinity complex and uncomplexed aptamer that has associated with the probe on the solid support is then partitioned from the remainder of the mixture, thereby removing free target and all other uncomplexed matter in the test sample (sample matrix); i.e., components of the mixture not associated with the first solid support. This partitioning step is referred to herein as the Catch-1 partition (see definition below). Following partitioning the aptamer-target affinity complex, along with any uncomplexed aptamer, is released from the first solid support using a method appropriate to the particular releasable first tag being employed.

In one embodiment, aptamer-target affinity complexes bound to the solid support are treated with an agent that introduces a second tag to the target molecule component of the aptamer-target affinity complexes. In one embodiment, the target is a protein or a peptide, and the target is biotinylated by treating it with NHS-PEO4-biotin. The second tag introduced to the target molecule may be the same as or different from the aptamer capture tag. If the second tag is the same as the first tag, or the aptamer capture tag, free capture sites on the first solid support may be blocked prior to the initiation of this tagging step. In this exemplary embodiment, the first solid support is washed with free biotin prior to the initiation of target tagging. Tagging methods, and in particular, tagging of targets such as peptides and proteins are described in U.S. Patent No. 7,855,054.

Partitioning is completed by releasing of uncomplexed aptamers and aptamer-target affinity complexes from the first solid support. In one embodiment, the first releasable tag is a photocleavable moiety that is cleaved by irradiation with a UV lamp under conditions that cleave ≥ 90% of the first releasable tag. In other embodiments, the release is accomplished by the method appropriate for the selected releasable moiety in the first releasable tag. Aptamer-target affinity complexes may be eluted and collected for further use in the assay or may be contacted to another solid support to conduct the remaining steps of the assay.

In one embodiment, a second partition is performed (referred to herein as the Catch-2 partition, see definition below) to remove free aptamer. As described above, in one embodiment, a second tag used in the Catch-2 partition may be added to the target while the aptamer-target affinity complex is still in contact with the solid support used in the Catch-0 capture. In other embodiments, the second tag may be added to the target at another point in the assay prior to initiation of Catch-2 partitioning. The mixture is contacted with a solid support, the solid support having a capture element (second) adhered to its surface which is capable of binding to the target capture tag (second tag), preferably with high affinity and specificity. In one embodiment, the solid support is magnetic beads (such as DynaBeads MyOne Streptavidin C1) contained within a well of a microtiter plate and the capture element (second capture element) is streptavidin. The magnetic beads provide a convenient method for the separation of partitioned components of the mixture. Aptamer-target affinity complexes contained in the mixture are thereby bound to the solid support through the binding interaction of the target (second) capture tag and the second capture element on the second solid support. The aptamer-target affinity complex is then partitioned from the remainder of the mixture, e.g. by washing the support with buffered solutions, including buffers comprising organic solvents including, but not limited to glycerol.

Aptamers are then selectively eluted from aptamer-target complexes with buffers comprising chaotropic salts from the group including, but not limited to sodium perchlorate, lithium chloride, sodium chloride and magnesium chloride. Aptamers retained on Catch-2 beads by virtue of aptamer/aptamer interaction are not eluted by this treatment.

In another embodiment, the aptamer released from the Catch-2 partition is detected and optionally quantified by any suitable nucleic acid detection methods, such as, for example, DNA microarray hybridization, Q-PCR, mass spectroscopy, the Invader assay, next-generation sequencing, and the like. These detection methods are described in further detail below.

Any of the methods described herein may be used to conduct a single-analyte test or a multiplexed analysis of a test sample. Any multiplexed analysis can include the use of two, tens, hundreds, or thousands of aptamers to simultaneously assay an equal number of target molecules in a test sample, such as a biological sample, for example. In these embodiments, a plurality of aptamers is introduced to the test sample and any of the above-described assays can be performed. After release of the aptamers, any suitable multiplexed nucleic acid detection methods can be employed to independently measure the different aptamers that have been released. In one embodiment, this can be accomplished by hybridization to complementary probes that are separately arranged on a solid surface. In another embodiment, each of the different aptamers may be detected based on molecular weight using mass spectroscopy. In yet another embodiment, each of the different aptamers can be detected based on electrophoretic mobility, such as, for example, in capillary electrophoresis, in a gel, or by liquid chromatography. In another embodiment, unique PCR probes can be used to detect and optionally quantify each of the different aptamers using Q-PCR. In another embodiment, next-generation sequencing methods can be used to detect and optionally quantify each of the different aptamers.

In each of the assays disclosed herein, a kinetic challenge may be used to increase the specificity of the assay and to reduce non-specific binding. In one embodiment, which can optionally be employed in each of the assays described herein, additional reduction in the non-specific binding may be accomplished by either pre-incubation of a competitor with the test sample or by addition of a competitor to the mixture during equilibrium binding. In other embodiments the kinetic challenge is performed by dilution.

Another embodiment describes a method for detecting a target molecule that may be present in a test sample, the method comprising: exposing an aptamer having a specific affinity for the target molecule and bearing a first tag having a specific affinity to a first capture element to a first solid support comprising a first capture element and allowing the first tag to associate with the first capture element; washing said solid support with one or more buffered solutions that dissociate aggregated aptamers; and eluting aptamers from said solid support with one or more buffered solutions comprising a chaotropic salt that disrupts aptamer/analyte interactions but supports aptamer/aptamer interactions and DNA hybridization. In one embodiment the chaotropic salt is selected from the group consisting of sodium perchlorate, lithium chloride, sodium chloride and magnesium chloride and the buffered solution that dissociates aggregated aptamers comprises an organic solvent. In one embodiment, the organic solvent is glycerol.

### BRIEF DESCRIPTION OF THE FIGURES

Figure 1 depicts graphically aptamer-dependent retention and subsequent elution of aptamers from Catch-2 beads. Radiolabeled aptamer bearing no biotin was incubated with magnetic streptavidin beads bearing increasing amounts of non-radiolabeled biotinylated aptamer and washed. Retained material was then eluted with 1 M sodium chloride in CAPS buffer at pH 10. The amount of radiolabeled aptamer eluted is proportional to the amount of cold aptamer adsorbed to Catch-2 beads.
Figures 2A-2F illustrate the effect of the concentration of blood plasma on the assay when the aptamer is not pre-immobilized prior to equilibration with test sample. With reference to Figure 2, plasma was titrated from 0% v/v to 25% v/v. As can be seen, the signal of most analytes plateaus between 10 and 20%.

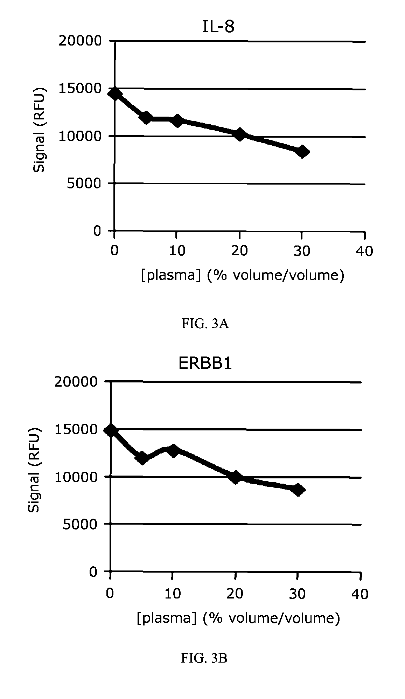

Figures 3A-3F depict graphically the recovery of aptamer in photocleavage eluate as a function of plasma concentration when aptamer is not pre-immobilized prior to equilibration with test sample. Catch-1 photocleavage (eluate was recovered and quantified by hybridization (Y-axis, relative fluorescence units). As can be seen aptamer recovery declines dramatically with increasing plasma concentration, with significant effects seen even at 5% plasma. It is unknown whether analyte binding affects aptamer binding to beads, however, it should be noted that preferential loss of complexed aptamers would generate even greater plasma-dependent effects.
Figures 4A-4D depict graphically a comparison of plasma titrations in standard (black curves) and pre-immobilized (dotted curves) assays.
Figures 5A-5D depict graphically a comparison of 1 M NaCl/CAPSO elution and 1.8 M NaClO₄/PIPES elution using the pre-immobilized assay format described herein. Standard curves in buffer (lower curves) and spikes in 40% plasma (upper curves) were run in pre-immobilized assay format.
Figure 6 depicts CV's (coefficients of variation) over 8 replicate buffer-only wells using perchlorate elution and pre-immobilized aptamers.
Figure 7 illustrates spike and recovery measured for 300 analytes. Spike recovery is defined as (analyte signal _{10 pM} spiked into ₚₗₐₛₘₐ - analyte signal ₚₗₐₛₘₐ)/analyte signal _{10 pM buffer spike}).
Figure 8 illustrates a left-shifted buffer dose-response in the immobilized format for the protein ERBB2. Measured endogenous levels are more than ten-fold lower and very near reported endogenous levels.
Figure 9 illustrates the improved protein titration in buffer, better spike and recovery behavior, more linear behavior of the plasma titration and more stable predicted endogenous protein levels in plasma for the protein Activin A.

### DETAILED DESCRIPTION

Reference will now be made in detail to representative embodiments of the invention. While the invention will be described in conjunction with the enumerated embodiments, it will be understood that the invention is not intended to be limited to those embodiments. On the contrary, the invention is intended to cover all alternatives, modifications, and equivalents that may be included within the scope of the present invention as defined by the claims.

The practice of the current invention employs, unless otherwise indicated, conventional methods of chemistry, microbiology, molecular biology, and recombinant DNA techniques within the level of skill in the art. Such techniques are explained fully in the literature. See, *e.g.,* Sambrook, et al. Molecular Cloning: A laboratory Manual (Current Edition); DNA Cloning: A Practical Approach, vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition).

Unless defined otherwise, technical and scientific terms used herein have the same meaning as commonly understood by one of ordinary skill in the art(s) to which this invention belongs. Although any methods, devices, and materials similar or equivalent to those described herein can be used in the practice or testing of the invention, the preferred methods, devices and materials are now described.

Examples in cited publications and limitations related therewith are intended to be illustrative and not exclusive. Other limitations of the cited publications will become apparent to those of skill in the art upon a reading of the specification and a study of the drawings.

The present disclosure includes methods, devices, reagents, and kits designed to improve the performance of multiplexed aptamer-based assays. The disclosed methods, devices, reagents, and kits provide high sensitivity assays for the detection and/or quantification of target molecules in a test sample by reducing or eliminating of background signal.

It is noteworthy that, unless otherwise specified in a particular embodiment, the methods for the detection and/or quantification of a target molecule described herein are independent of the specific order in which the steps are described. For purposes of illustration, the methods are described as a specific sequence of steps; however, it is to be understood that any number of permutations of the specified sequence of steps is possible, so long as the objective of the particular assay being described is accomplished. Stated another way, the steps recited in any of the disclosed methods may be performed in any feasible order, and the methods of the invention are not limited to any particular order presented in any of the described embodiments, the examples, or the appended claims. Further, for convenience and ease of presentation, the various methods are described with reference to a single target molecule and a single aptamer. However, it is to be understood that any of the described methods can be performed in a multiplex format that can provide for the simultaneous detection and/or quantification of multiple targets using multiple aptamers, such that, for example, multiple target molecules in a test sample can be detected and/or quantified by contacting the test sample with multiple aptamers, wherein each aptamer has a specific affinity for a particular target molecule (i.e., in a multiplex format).

As used in this disclosure, including the appended claims, the singular forms "a," "an," and "the" include plural references, unless the content clearly dictates otherwise, and are used interchangeably with "at least one" and "one or more." Thus, reference to "an aptamer" includes mixtures of aptamers, and the like.

As used herein, the term "about" represents an insignificant modification or variation of the numerical value such that the basic function of the item to which the numerical value relates is unchanged.

The term "each" when used herein to refer to a plurality of items is intended to refer to at least two of the items. It need not require that all of the items forming the plurality satisfy an associated additional limitation.

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but may include other elements not expressly listed or inherent to such process, method, product-by-process, or composition of matter.

As used herein, "associate," "associates," and any variation thereof refers to an interaction or complexation between a tag and a probe resulting in a sufficiently stable complex so as to permit separation of "unassociated" or unbound materials, such as, for example, unbound components of a test sample, from the tag-probe complex under given complexation or reaction conditions. A tag and a probe can associate with each other directly by interacting and binding to each other with specificity. A tag and a probe can also associate with each other indirectly such as when their complexation is mediated by a linker molecule.

As used herein, the term "nucleotide" refers to a ribonucleotide or a deoxyribonucleotide, or a modified form thereof, as well as an analog thereof. Nucleotides include species that include purines (*e.g*., adenine, hypoxanthine, guanine, and their derivatives and analogs) as well as pyrimidines (*e.g.*, cytosine, uracil, thymine, and their derivatives and analogs).

As used herein, "nucleic acid," "oligonucleotide," and "polynucleotide" are used interchangeably to refer to a polymer of nucleotides and include DNA, RNA, DNA/RNA hybrids and modifications of these kinds of nucleic acids, oligonucleotides and polynucleotides, wherein the attachment of various entities or moieties to the nucleotide units at any position are included. The terms "polynucleotide," "oligonucleotide," and "nucleic acid" include double- or single-stranded molecules as well as multi-stranded (i.e., triple-helical) molecules. Nucleic acid, oligonucleotide, and polynucleotide are broader terms than the term aptamer and, thus, the terms nucleic acid, oligonucleotide, and polynucleotide include polymers of nucleotides that are aptamers but the terms nucleic acid, oligonucleotide, and polynucleotide are not limited to aptamers.

As used herein, "nucleic acid ligand," "aptamer," "SOMAmer" and "clone" are used interchangeably to refer to a non-naturally occurring nucleic acid that has a desirable action on a target molecule. A desirable action includes, but is not limited to, binding of the target, catalytically changing the target, reacting with the target in a way that modifies or alters the target or the functional activity of the target, covalently attaching to the target (as in a suicide inhibitor), and facilitating the reaction between the target and another molecule. In one embodiment, the action is specific binding affinity for a target molecule, such target molecule being a three dimensional chemical structure other than a polynucleotide that binds to the nucleic acid ligand through a mechanism which is independent of Watson/Crick base pairing or triple helix formation, wherein the aptamer is not a nucleic acid having the known physiological function of being bound by the target molecule. Aptamers to a given target include nucleic acids that are identified from a candidate mixture of nucleic acids, where the aptamer is a ligand of the target, by a method comprising: (a) contacting the candidate mixture with the target, wherein nucleic acids having an increased affinity to the target relative to other nucleic acids in the candidate mixture can be partitioned from the remainder of the candidate mixture; (b) partitioning the increased affinity nucleic acids from the remainder of the candidate mixture; and (c) amplifying the increased affinity nucleic acids to yield a ligand-enriched mixture of nucleic acids, whereby aptamers of the target molecule are identified. It is recognized that affinity interactions are a matter of degree; however, in this context, the "specific binding affinity" of an aptamer for its target means that the aptamer binds to its target with a much higher degree of affinity than it binds to other, non-target, components in a mixture or sample. An aptamer can include any suitable number of nucleotides. "Aptamers" refer to more than one such set of molecules. Different aptamers can have either the same or different numbers of nucleotides. Aptamers may be DNA or RNA and may be single stranded, double stranded, or contain double stranded or triple stranded regions. Aptamers may be designed with any combination of the base modified nucleotides desired.

As used herein, a "SOMAmer" or Slow Off-Rate Modified Aptamer refers to an aptamer (including an aptamers comprising at least one nucleotide with a hydrophobic modification) with an off-rate (t_{½}) of ≥ 30 minutes. In some embodiments, SOMAmers are generated using the improved SELEX methods described in U.S. Patent 7,947,447, entitled "Method for Generating Aptamers with Improved Off-Rates."

An aptamer can be identified using any known method, including the SELEX process. See, *e.g.,* U.S. Patent No. 5,475,096 entitled "Nucleic Acid Ligands". Once identified, an aptamer can be prepared or synthesized in accordance with any known method, including chemical synthetic methods and enzymatic synthetic methods.

As used herein, the terms "aptamer-target affinity complex", "aptamer affinity complex" or "aptamer complex" refer to a non-covalent complex that is formed by the interaction of an aptamer with its target molecule. "Aptamer-target affinity complexes", "aptamer affinity complexes" or "aptamer complexes" refer to more than one such set of complexes. An aptamer-target affinity complex, aptamer affinity complex or aptamer complex can generally be reversed or dissociated by a change in an environmental condition, *e.g.,* an increase in temperature, an increase in salt concentration, or an addition of a denaturant.

In some embodiments, a non-covalent complex of an aptamer and its target is provided, wherein the aptamer has a K_{d} for the target of about 100 nM or less, wherein the rate of dissociation (as given by half-life of the complex; t_{1/2}) of the aptamer from the target is greater than or equal to about 30 minutes; and/or wherein one, several or all pyrimidines in the nucleic acid sequence of the aptamer are modified at the 5-position of the base.

As used herein, "non-specific complex" refers to a non-covalent association between two or more molecules other than an aptamer and its target molecule. Because a non-specific complex is not selected on the basis of an affinity interaction between its constituent molecules, but represents an interaction between classes of molecules, molecules associated in a non-specific complex will exhibit, on average, much lower affinities for each other and will have a correspondingly higher dissociation rate than an aptamer and its target molecule. Non-specific complexes include complexes formed between an aptamer and a non-target molecule, an aptamer and another aptamer, a competitor and a non-target molecule, a competitor and a target molecule, an aptamer and a competitor, and a target molecule and a non-target molecule as well as higher order aggregates of aptamer, target molecule, non-target molecule, surface and competitor.

As used herein, "target molecule," "analyte," and "target" are used interchangeably to refer to any molecule of interest to which an aptamer can bind with high affinity and specificity and that may be present in a test sample. A "molecule of interest" includes any minor variation of a particular molecule, such as, in the case of a protein, for example, minor variations in amino acid sequence, disulfide bond formation, glycosylation, lipidation, acetylation, phosphorylation, or any other manipulation or modification, such as conjugation with a labeling component that does not substantially alter the identity of the molecule. Exemplary target molecules include proteins, polypeptides, nucleic acids, carbohydrates, lipids, polysaccharides, glycoproteins, hormones, receptors, antigens, antibodies, affibodies, antibody mimics, viruses, pathogens, toxic substances, substrates, metabolites, transition state analogs, cofactors, inhibitors, drugs, dyes, nutrients, growth factors, cells, tissues, and any fragment or portion of any of the foregoing. An aptamer may be identified for virtually any chemical or biological molecule of any size, and thus virtually any chemical or biological molecule of any size can be a suitable target. A target can also be modified to enhance the likelihood or strength of an interaction between the target and the aptamer. A target can also be modified to include a tag, as defined above. In exemplary embodiments, the target molecule is a protein. See U.S. Patent No. 6,376,190 entitled "Modified SELEX Processes Without Purified Protein" for methods in which the SELEX target is a peptide.

"Polypeptide," "peptide," and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. Polypeptides can be single chains or associated chains.

The term "test sample" refers herein to any material, solution, or mixture that contains a plurality of molecules and may include at least one target molecule. The term test sample includes biological samples, as defined below, and samples that may be used for environmental or toxicology testing, such as contaminated or potentially contaminated water and industrial effluents, for example. A test sample may also be an end product, intermediate product, or by-product of a preparatory process, for example a manufacturing process. A test sample may include any suitable assay medium, buffer, or diluent that has been added to a material, solution, or mixture obtained from an organism or from some other source (*e.g.,* the environment or an industrial source).

The term "biological sample" refers to any material, solution, or mixture obtained from an organism. This includes blood (including whole blood, leukocytes, peripheral blood mononuclear cells, plasma, and serum), sputum, breath, urine, semen, saliva, meningeal fluid, amniotic fluid, glandular fluid, lymph fluid, nipple aspirate, bronchial aspirate, synovial fluid, joint aspirate, cells, a cellular extract, and cerebrospinal fluid. This also includes experimentally separated fractions of all of the preceding. The term "biological sample" also includes materials, solutions, or mixtures containing homogenized solid material, such as from a stool sample, a tissue sample, or a tissue biopsy, for example. The term "biological sample" also includes materials, solutions, or mixtures derived from a cell line, tissue culture, cell culture, bacterial culture, viral culture or cell free biological system (e.g. IVTT).

In any of the embodiments disclosed herein, a test sample may be compared to a reference sample. A "reference sample" refers herein to any material, solution, or mixture that contains a plurality of molecules and is known to include at least one target molecule. The precise amount or concentration of any target molecules present in the reference sample may also be known. The term reference sample includes biological samples, as defined herein, and samples that may be used for environmental or toxicology testing, such as contaminated or potentially contaminated water and industrial effluents, for example. A reference sample may also be an end product, intermediate product, or by-product of a preparatory process, for example a manufacturing process. A reference sample may include any suitable assay medium, buffer, or diluent that has been added to a material, solution, or mixture obtained from an organism or from some other source (*e.g.,* the environment or an industrial source).

As used herein, "non-target molecule" and "non-target" are used interchangeably to refer to a molecule contained in a test sample that can form a non-specific complex with an aptamer. It will be appreciated that a molecule that is a non-target for a first aptamer may be a target for a second aptamer. Likewise, a molecule that is a target for the first aptamer may be a non-target for the second aptamer.

As used herein, the term "partition" refers to a separation, concentration or removal of one or more molecular species from the test sample or other molecules in the test sample. Partitioning can be used to increase sensitivity and/or reduce background. Partitioning is most effective following aptamer complex formation or when the aptamer-target affinity complex becomes irreversible due to the covalent bond introduced during crosslinking. A partitioning step may be introduced after any step, or after every step, where the aptamer-target affinity complex is immobilized. Partitioning may also rely on a size differential or other specific property that differentially exists between the aptamer-target affinity complex and other components of the test sample. Partitioning may also be achieved through a specific interaction with an aptamer or target. Partitioning maybe also be accomplished based on the physical or biochemical properties of the aptamer, target, aptamer-target affinity complex or aptamer-target covalent complex.

In single analyte and multiplexed aptamer assays, a number of steps have been designed to separate specific aptamer/affinity complexes from materials in the sample or assay reagents that may lead to confounding background signals. Despite implementing these steps, background remains an issue in these types of assays. Background in an analytical method can be addressed empirically or a better approach is to identify the source of the background and eliminate the interaction that leads to background. It has been discovered that aptamer-aptamer interaction is a source of background in multiplexed aptamer methods. Reagents that reduce DNA-DNA and RNA-RNA interactions, including those that are sequence based are known. Because the internal interactions of an aptamer determines the aptamer's secondary and tertiary structure, these types of reagents would not have been expected to substantially reduce the background signal in a multiplexed assay without affecting aptamer folding and therefore, binding to its corresponding target. Materials and methods that balance the need to reduce background with the maintenance of aptamer structure are described.

The present disclosure describes improved methods to perform aptamer- and photoaptamer-based multiplexed assays for the quantification of one or more target molecule(s) that may be present in a test sample wherein the aptamer (or photoaptamer) can be separated from the aptamer-target affinity complex (or photoaptamer-target covalent complex) for final detection using any suitable nucleic acid detection method in as much as the materials and methods described herein can be used to improve overall assay performance. Photoaptamers are aptamers that comprise photoreactive functional groups that enable the aptamers to covalently bind or "photocrosslink" their target molecules.

Two unanticipated limitations emerged from detailed examination of prior described methods for performing single- and multi-plex aptamer based assays, including multiplexed proteomic aptamer affinity assays. First, aptamer/aptamer interactions were identified as a primary source of assay background and a potential limitation to multiplex capacity. Second, sample matrices (primarily serum and plasma) were found to inhibit the immobilization of biotinylated aptamers on streptavidin-substituted matrices. Three primary innovations are described herein which reduce and/or eliminate both of these limitations in the process. Two are unique to an improved method described herein (referred to herein as "Version 3" of the multiplexed assay; one was implemented in an earlier version of the assay as described in Gold et al. (Dec. 2010) "Aptamer-based multiplexed proteomic technology for biomarker discovery," PLoS One 5(12):e15005). Version 3 is one embodiment of the invention described herein.

The first improvement in the assay, as described in Gold et al. (PLoS One (2010) 5(12):e15005), comprised the use of organic solvents in some of the wash buffers of the Catch-2 step to diminish the dielectric constant of the medium. Addition of these wash buffers effectively accented the like-charge repulsion of adjacent phosphodiester backbones of the aptamers, thus promoting dissociation of background-causing interacting aptamers.

The second improvement in the process as described herein provides a dual advantage. First, as in the case of the addition of organic solvents to some of wash buffers used in the Catch-2 step of the assay, it also counters the tendency of aptamers to interact, and thus diminishes background and increases multiplex capacity. However, its primary advantage is to counteract the matrix-dependent inhibition of biotinylated aptamer adsorption to streptavidin matrices. Such inhibition is easily detectable even at 5% v/v plasma or serum, and limits working assay concentrations to 5-10% plasma or serum concentrations. This limitation in turn limits assay sensitivity.

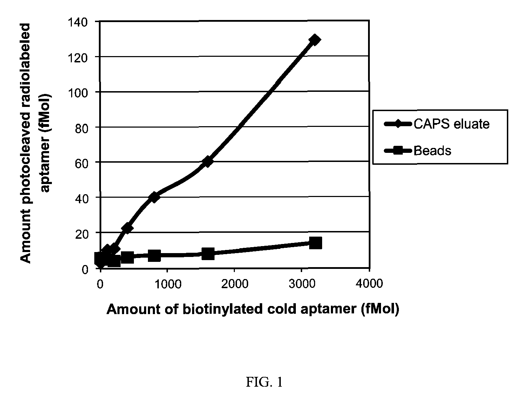

The second improvement to the multiplexed assay, as described herein comprises pre-immobilization of the tagged aptamers on the solid support matrices prior to equilibration (termed "Catch-0") with the test solution. Equilibration with the test solution is then carried out with bound aptamers, in the processing vessels themselves. As described herein for purposes of illustration only, biotinylated aptamers were pre-immobilized on streptavidin bead matrices, and equilibration with test solution carried out with the bead-bound aptamers. This pre-immobilization step enables immobilization under conditions where aptamers have diminished tendency to interact and also enables very stringent washes (with base and with chaotropic salts) prior to equilibration, disrupting interacting aptamers and removing all aptamers not bound through the very robust biotin-streptavidin interaction. This reduces the number of aptamer "clumps" traversing the assay - clumps that have at some detectable frequency retained the biotin moiety or become biotinylated in the assay. It is worth noting that irradiation cleaves most, but not all photocleavable biotin moieties from aptamers, while some aptamers become biotinylated via the NHS-biotin treatment intended to "tag" proteins. Biotinylated aptamer that is captured at the Catch-2 step creates background by interacting with bulk photocleaved aptamer, which is then released upon elution (Figure 1). It should also be noted that a pre-immobilized format will likely support very high multiplex capacities as aptamer panels may be immobilized separately then combined in bead-bound form, thus bypassing conditions in which aptamers may interact and clump.

Thus, pre-immobilization bypasses the need for aptamer adsorption in the presence of analyte solution, thus ensuring quantitative immobilization even when assaying inhibitory concentrations of analyte solutions. This enables the use of much higher concentrations, up to and including at least 40% v/v plasma or serum, rather than the 10% top concentration of the process as previously described (Gold et al. (Dec. 2010) PLoS One 5(12):e15005) or the 5% top concentration used in more recent editions of the process thereby increasing sensitivity roughly 4- to 8-fold, as well as, increasing the overall robustness of the assay.

The third improvement to the overall process, as described herein comprises the use of a chaotropic salt at neutral pH for elution during the Catch-2 step as described in detail below. Prior methods comprised the use of sodium chloride at high pH (10), which disrupts DNA hybridization and aptamer/aptamer interaction as well as protein/aptamer interaction. As noted above, DNA hybridization and aptamer/aptamer interactions contribute to assay background. Chaotropic salts, including but not limited to sodium perchlorate, lithium chloride, sodium chloride and magnesium chloride at neutral pH, support DNA hybridization and aptamer/aptamer interactions, while disrupting aptamer/protein interactions. The net result is significantly diminished (about 10-fold) background, with a concomitant rise in assay sensitivity.

### Overview of Prior Described Multiplex Aptamer Assays

Aptamers were equilibrated with a test sample (e.g. plasma) in solution. Long-lived (median half-life circa 30 minutes) complexes between analytes and aptamers, particularly slow off-rate aptamers were formed in this period. The equilibration mixture, comprising the sample matrix, aptamer, protein analytes, and aptamer/analyte complexes was then exposed to streptavidin immobilized on agarose beads (SA-agarose) (in the case in which the aptamer is tagged with a biotin moiety). Aptamers in the mixture are captured on the SA-agarose via the appended biotin moiety. Note that the assay is dependent on quantitative aptamer capture at this step. The immobilized aptamers were then washed under mild conditions, removing free and loosely bound protein, but leaving aptamer and aptamer/analyte complexes behind. This step, termed "Catch-1", can be thought of as a protein purification step. (See e.g., U.S. Patent Nos. 7,947,447 and 7,855,054).

The agarose beads, bearing aptamers and analyte/aptamer complexes, were then treated with NHS-biotin, leaving a biotin "tag" on the aptamer-bound protein analytes. After further washing, aptamers, including aptamer/biotinylated analyte complexes were released from the agarose beads via cleavage of a labile linker between the aptamer and biotin moiety (as described in the example below for purposes of illustration only, a photolabile linker that is cleaved upon exposure to UV light is used). The so-called photocleavage eluate is then transferred to a well bearing magnetic streptavidin beads. Aptamer/biotinylated analyte complexes are preferentially adsorbed. This capture step is referred to as "Catch-2". (See e.g., U.S. Patent Nos. 7,947,447 and 7,855). After washes, aptamers which are now bound to beads through analytes were eluted with a sodium chloride solution at elevated pH. Recovered aptamers were then quantified by hybridization to commercial microarrays. Recovered aptamer amounts serve as a surrogate for protein concentration, which are formally determined by means of a standard curve.

As noted above, it had been observed that substantial amounts of aptamer traverse the assay (i.e. pass through the partitioning steps) and create background even in the absence of added protein from a test sample. The source of this protein-independent background was traced to aptamer/aptamer interaction, as illustrated in Figure 1. Several mitigation strategies were explored to address this issue, which are described in Gold et al. (PLoS One (2010) 5(12):e15005). Ultimately, warm glycerol washes were selected as an effective and suitable strategy to mitigate this issue problem. However, other solvents and reagents that reduce the dielectric constant of water are capable of mitigating assay background in a similar way. Examples include, but are not limited to glycerol, propylene glycol, trehalose, ethanol and the like.

### Mitigation of Capture Inhibition by Pre-Absorption of Aptamers

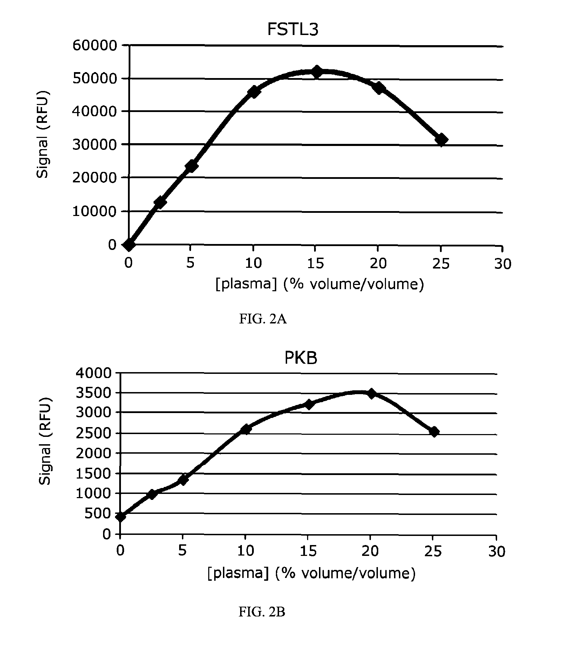

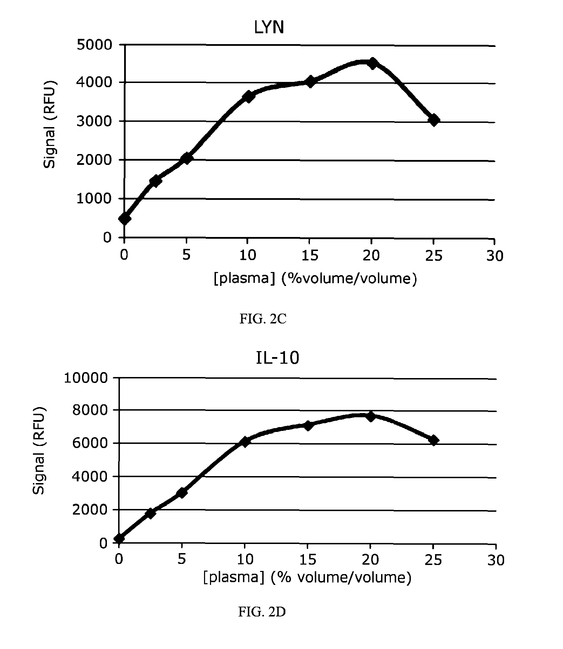

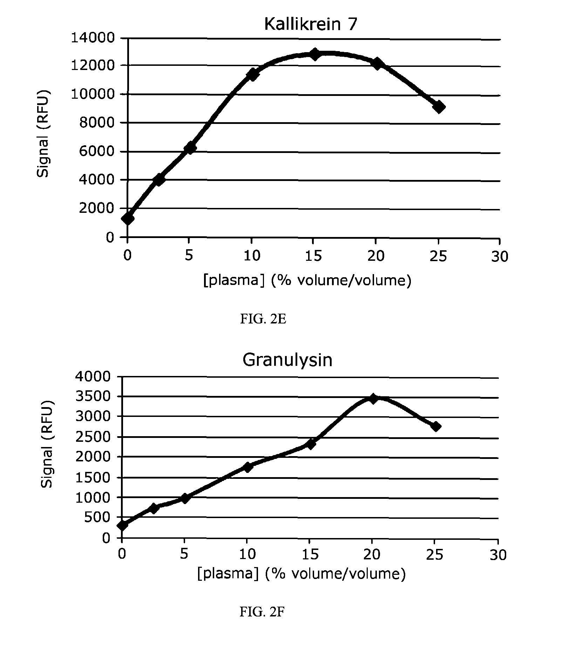

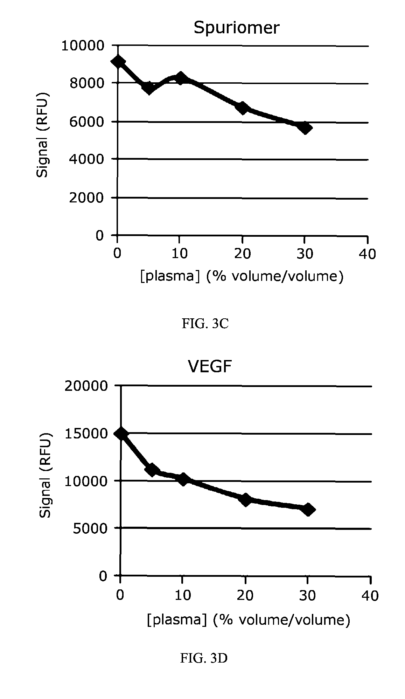

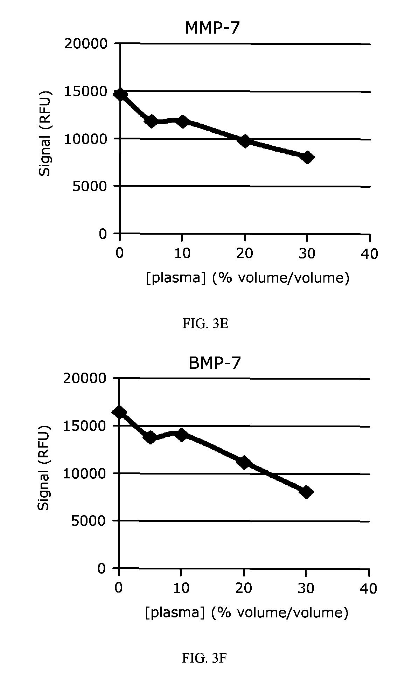

As noted above, it was determined that serum and plasma diminishes capture efficiency of aptamers, limiting the concentration that can be measured as illustrated in Figures 2 and 3.

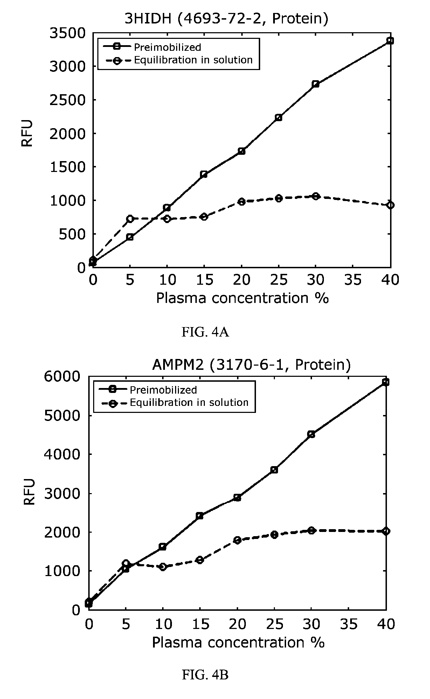

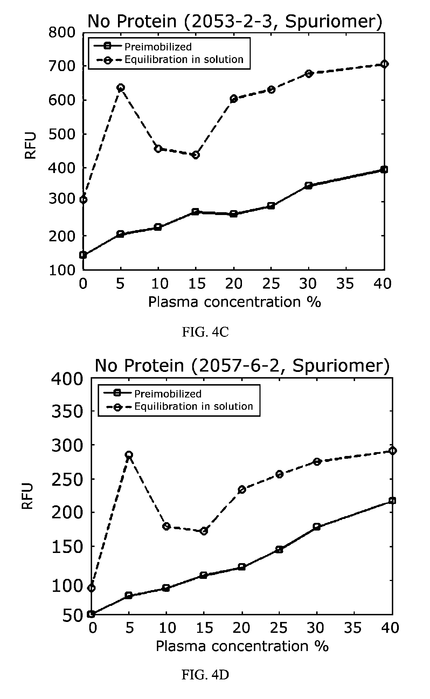

As illustrated in Figure 4, pre-immobilization of aptamers and subsequent equilibration, as described herein, mitigates the problem of capture inhibition and enables the use of much high plasma concentrations. Specifically, concentrations up to and including at least 40% v/v plasma or serum, rather than the 10% top concentration of the process as previously described in Gold et al. (PloS One (Dec.2010) 5(12):e15005), or the 5% top concentration of more recent editions may be used, thereby increasing sensitivity roughly 4-to 8-fold, as well as, increasing the overall robustness of the assay.

With reference to Figure 4, it can be seen that a linear increase in signal can be observed all the way up to 40% v/v plasma (compare line marked with (□), pre-immobilized format, to line marked with (○), standard format. Note that a signal observed for a Spuriomer (a surrogate for protein-dependent background, bottom two panels) is considerably higher in the standard format, suggesting that pre-immobilization can reduce protein-dependent background.

### Reduction in Assay Background and Increase in Assay Sensitivity Using a Chaotropic Salt for Elution

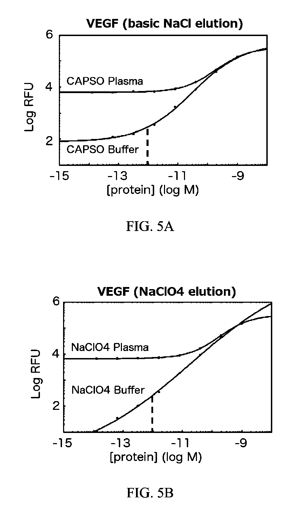

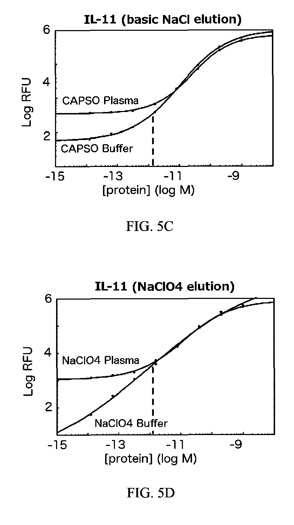

As illustrated in Figure 5, the use of a chaotropic salt for elution both diminishes assay background and increases assay sensitivity. With reference to Figure 5, it can be seen that assay background in buffer is significantly reduced with perchlorate elution (compare the lower curve in Figure 5A with the lower curve in Figure 5B). Assay background has dropped to 20-40 RFU. Apparent endogenous levels are also somewhat reduced for IL-11 with perchlorate elution, indicating diminished protein-dependent background (roughly 0.2 pM vs. 1 pM).

The following table summarizes the results (RFU) of the comparison of CAPSO/NaCl elution and perchlorate elution. Median and average signals for all SOMAmers in each dilution group (column 2) in the presence of plasma (5% v/v plasma for 5% SOMAmers; 0.316% v/v plasma for 0.316% SOMAmers, and 0.01% plasma for 0.01% SOMAmers) are shown in rows 1-6; median and average signals for all SOMAmers termed Spuriomers (SOMAmers designed in silico that do not have a cognate analyte) in the presence of 5% plasma are shown in rows 8 and 9; and median and average signals of all SOMAmers in all dilutions in the presence of buffer only are shown in row 10.

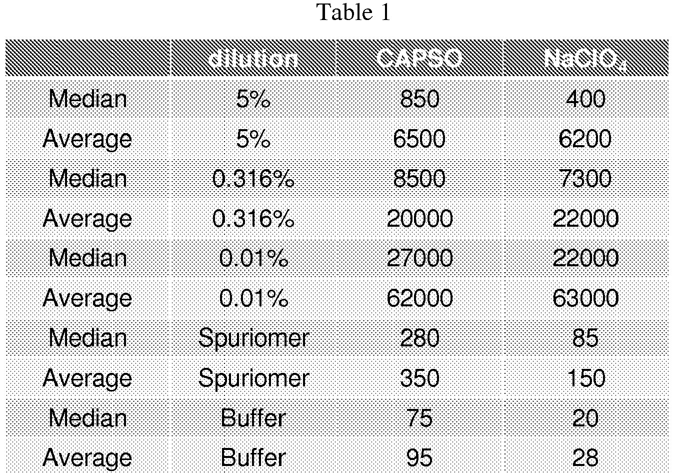

The results depicted in Figure 5 are mirrored in the results shown in the table. Perchlorate elution generates comparatively low buffer signals, significantly reduced Spuriomer signals in plasma, and marginally reduced signals in the 0.01% and 0.316% mixes where signals unambiguously originate from cognate analytes.

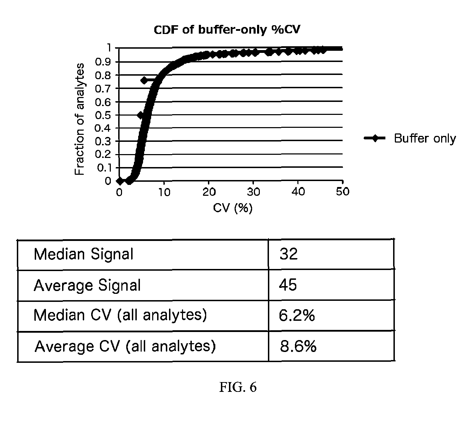

Coefficients of variation (CV's) in buffer signals with perchlorate elution were measured over 8 replicates, reasoning that signals close to machine background were entitled to be noisy, and thus could preclude realization of the very low lower limits of quantification (LLOQ) suggested by these very low backgrounds. In this experiment, with a median signal of just 32 RFU, the raw median CV was 6.2% (Figure 6). Stability at these very low signals was not likely to be a factor that limits LLOQ.

### Comparison of assay performance

A formal comparison between the most recent version of the method disclosed in Gold et al. (PLoS One (Dec. 2010) 5(12):e15005) (termed "Previous assay" in Table 2) and the instant disclosure (Version 3 in Table 2) was made. The high degree of similarity between protocols permitted a test in which both assay protocols could be run in a single robotic run, in fact on the same filter plates, with minimal manual intervention. The test included 8 replicate plasma samples, to determine variability, 8 buffer dose-response wells, and 8 plasma spike wells. Note that Version 3 used much greater plasma concentrations than an earlier assay version did the previous assay (40, 1.3, and 0.044% as opposed to 5, 0.167, and 0.0056%). A summary of the results in RFU space and in RFU normalized to plasma concentration is shown below (Table 2).

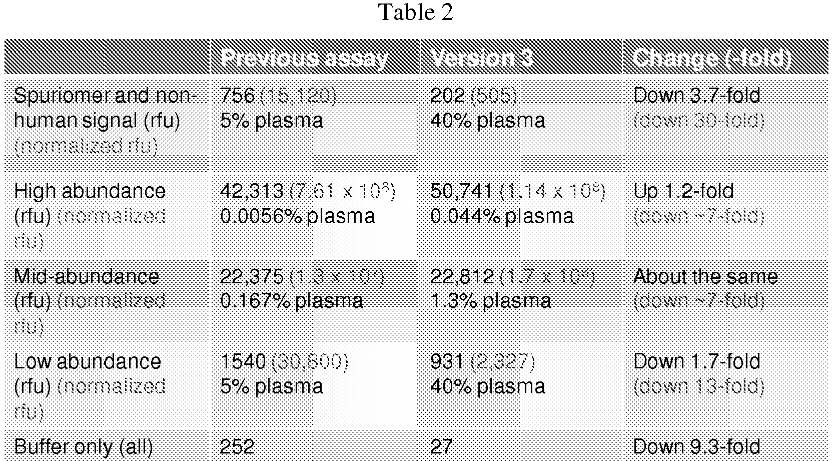

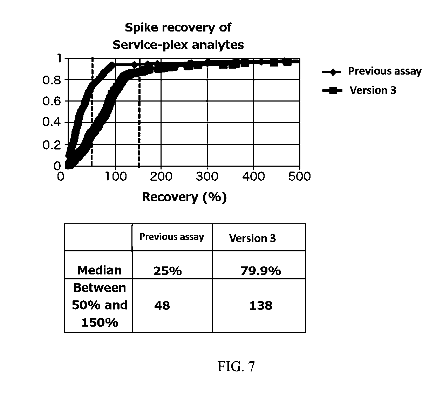

With reference to Table 2, it can be seen that raw background signals are diminished in Version 3 by about 4-fold as determined by signals from Spuriomer and aptamers for non-human targets, while raw analyte signals are about the same, as measured by signals from mid- and high-abundance analytes. Note that these values were obtained with 40% plasma in Version 3, while 5% plasma was used in an earlier assay version. If one normalizes signals to 100% plasma (values in parentheses), backgrounds drop significantly (by about 30-fold) while analyte signals are down about 7-fold. Buffer-only signals drop by nearly a log. Note that the reduction in background comes at a cost - about 15 µL of plasma are required for the previous assay, while about 60 µL are required for the Version 3. This elevated sample consumption is likely unimportant for large-animal samples (e.g. human), but may become a factor for analysis of longitudinal samples for small animals such as mice. Overall spike recoveries were much higher for the embodiment of the present invention demonstrated in Version 3, than for the earlier assay version, with medians running about 80% for the and just 25% for the previous assay, as illustrated in Figure 7. Much of this improvement can be attributed to immobilization of the aptamers prior to equilibration.

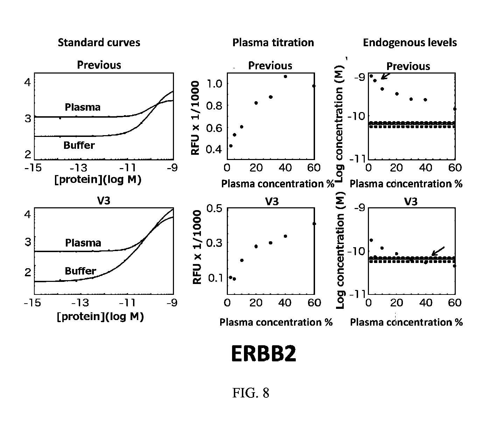

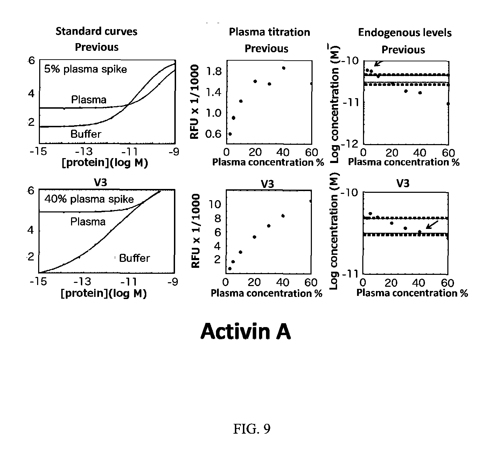

Figures 8 and 9 depict two examples of a direct comparison of protein titration curves in buffer (left panels, lower curve), protein spikes into plasma (left panels, upper curve), plasma titration (middle panels) and calculated endogenous levels (mapping of plasma titration to protein standard curves, right panels) for the previous assay (top panels) and the method described herein (bottom panels). Note that the protein was spiked in 5% plasma for the previous assay and 40% plasma for the method described herein. A typical comparison curve showing buffer dose-response, plasma spike, and measured endogenous levels can be seen in Figure 8. A clear example of improved spike recovery can be seen in Figure 9.

### Improved Multiplexed Aptamer Assay

In one embodiment, aptamers are provided that have high affinity and specificity for a target molecule and a first releasable tag. In some embodiments the aptamers are photoaptamers. In another embodiment, the first releasable tag is added at any time in the assay prior to the Catch-1 (as defined below in paragraph [0082]) partition. In one embodiment, this first releasable tag is a photocleavable biotin. Other tags and cleavable moieties and aptamer containing such tags and cleavable moieties are described.

The aptamer comprising the releasable first tag that has a specific affinity for a target molecule is immobilized on a solid support in solution prior to equilibration with the test sample. The attachment of the aptamer to the solid support is accomplished by contacting a first solid support with the aptamer and allowing the releasable first tag included on the aptamer to associate, either directly or indirectly, with an appropriate first capture agent that is attached to the first solid support. Washes with a solution buffered to pH 11 remove aptamer/aptamer aggregates, thereby reducing assay background. These steps comprise "Catch-0".

A test sample is then prepared (as described in the Example) and contacted with the immobilized aptamers that have a specific affinity for their respective target molecules. If the test sample contains the target molecule(s), an aptamer-target affinity complex will form in the mixture with the test sample. Note that in addition to aptamer-target affinity complexes, uncomplexed aptamer will also be attached to the first solid support. The aptamer-target affinity complex and uncomplexed aptamer that has associated with the solid support is then partitioned from the remainder of the mixture, thereby removing free target and all other uncomplexed matter in the test sample (sample matrix); i.e., components of the mixture not associated with the first solid support. Following partitioning the aptamer-target affinity complex, along with any uncomplexed aptamer, is released from the first solid support using a method appropriate to the particular releasable first tag being employed.

In one embodiment, aptamer-target affinity complexes bound to the solid support are then treated with an agent that introduces a second tag to the target molecule component of the aptamer-target affinity complexes. In one embodiment, the target is a protein or a peptide, and the target is biotinylated by treating it with NHS-PEO4-biotin. The second tag introduced to the target molecule may be the same as or different from the aptamer capture tag. If the second tag is the same as the first tag, or the aptamer capture tag, free capture sites on the first solid support may be blocked prior to the initiation of this tagging step. In this exemplary embodiment, the first solid support is washed with free biotin prior to the initiation of target tagging. Tagging methods, and in particular, tagging of targets such as peptides and proteins are described in U.S. Patent No. 7,855,054. In other embodiments, tagging of the target is performed at any other point in the assay prior to initiation of the Catch-2 partitioning.

Catch-1 partitioning is completed by releasing of aptamers and aptamer-target affinity complexes from the first solid support. In one embodiment, the first releasable tag is a photocleavable moiety that is cleaved by irradiation with a UV lamp under conditions that cleave ≥ 90% of the first releasable tag. In other embodiments, the release is accomplished by the method appropriate for the selected releasable moiety in the first releasable tag. Aptamer-target affinity complexes may be eluted and collected for further use in the assay or may be contacted with another solid support to conduct the remaining steps of the assay.

In one embodiment, the mixture may optionally be subject to a kinetic challenge. The kinetic challenge helps reduce any non-specific binding between aptamers and non-target molecules. In one embodiment, 10 mM dextran sulfate is added to the aptamer-target affinity complexes, and the mixture is incubated for about 15 minutes. Other competitors include but are not limited to competitor nucleic acids. In another embodiment, the kinetic challenge is initiated by performing the Catch-1 elution in the presence of 10 mM dextran sulfate. In other embodiments, the kinetic challenge is performed after the equilibrium binding step and before the Catch-2 partitioning. In other embodiments the kinetic challenge is performed by dilution.

In one embodiment, the Catch-2 partition is performed to remove free aptamer. As described above, in one embodiment, a second tag used in the Catch-2 partition may be added to the target while the aptamer-target affinity complex is still in contact with the solid support used in the Catch-1 partition. In other embodiments, the second tag may be added to the target at another point in the assay prior to initiation of Catch-2 partitioning. The mixture is contacted with a solid support, the solid support having a capture element (second) adhered to its surface which is capable of binding to the target capture tag (second tag), preferably with high affinity and specificity. In one embodiment, the solid support is magnetic beads (such as DynaBeads MyOne Streptavidin C1) contained within a well of a microtiter plate and the capture element (second capture element) is streptavidin. The magnetic beads provide a convenient method for the separation of partitioned components of the mixture. Aptamer-target affinity complexes contained in the mixture are thereby bound to the solid support through the binding interaction of the target (second) capture tag and the second capture element on the second solid support. The aptamer-target affinity complex is then partitioned from the remainder of the mixture, e.g. by washing the support with buffered solutions, including buffers comprising organic solvents including but not limited to glycerol.

Aptamers are then selectively eluted from aptamer-target complexes with buffers comprising chaotropic salts from the group including but not limited to sodium perchlorate and lithium chloride. Aptamers retained on Catch-2 beads by virtue of aptamer/aptamer interaction are not eluted by this treatment.

In another embodiment, the aptamer released from the Catch-2 partition is detected and optionally quantified by any suitable nucleic acid detection methods, such as, for example DNA microarray hybridization, Q-PCR, mass spectroscopy, the Invader assay, next generation sequencing, and the like. These detection methods are described in further detail below.

In one embodiment, the reference sample can be a pooled biological sample that represents a control group. In another embodiment, the reference sample can be a biological sample obtained from an individual, collected at a first time, and the test sample can be obtained from the same individual but collected at a second time, thereby facilitating a longitudinal study of an individual by measuring and evaluating any changes in the amount or concentration of one or more target molecules in multiple biological samples provided by the individual over time.

Any of the methods described herein may be used to conduct a single-analyte test or a multiplexed analysis of a test sample. Any multiplexed analysis can include the use of two, tens, hundreds, or thousands of aptamers to simultaneously assay an equal number of target molecules in a test sample, such as a biological sample, for example. In these embodiments, a plurality of aptamers, each of which recognizes and optionally crosslinks to a different analyte, is introduced to the test sample and any of the above-described assays can be performed. After release of the aptamers, any suitable multiplexed nucleic acid detection methods can be employed to measure the different aptamers that have been released. In one embodiment, this can be accomplished by hybridization to complementary probes that are separately arranged on a solid surface. In another embodiment, each of the different aptamers may be detected based on molecular weight using mass spectroscopy. In yet another embodiment, each of the different aptamers can be detected based on electrophoretic mobility, such as, for example, in capillary electrophoresis, in a gel, or by liquid chromatography. In another embodiment, unique PCR probes can be used to quantify each of the different aptamers using Q-PCR.

In each of the assays disclosed herein, a kinetic challenge may be used to increase the specificity of the assay and to reduce non-specific binding. In one embodiment, which can optionally be employed in each of the assays described herein, additional reduction in the non-specific binding may be accomplished by either pre-incubation of a competitor with the test sample or by addition of a competitor to the mixture during equilibrium binding. In one embodiment, 4 µM of a Z-block competitor oligonucleotide (5'-(ACZZ)₇AC-3', where Z = 5-benzyl-dUTP) is preincubated for about 5 minutes with the test mixture.

### Kits

Another aspect of the present disclosure relates to kits useful for conveniently performing any of the methods disclosed herein to analyze test samples. To enhance the versatility of the disclosed methods, the reagents can be provided in packaged combination, in the same or separate containers, so that the ratio of the reagents provides for substantial optimization of the method and assay. The reagents may each be in separate containers or various reagents can be combined in one or more containers depending upon the cross-reactivity and stability of the reagents.

A kit comprises, in packaged combination, at least one tagged aptamer and one or more solid supports, each including at least one capture agent. The kit may also include washing solutions such as buffered aqueous medium for sample dilution as well as array washing, sample preparation reagents, and so forth. The kit may further contain reagents useful in introducing a second tag, generally through modification or derivatization of the target. In addition the kit may contain reagents suitable for performing the desired kinetic challenge during the analytical method. The relative amounts of the various reagents in the kits can be varied widely to provide for concentrations of the reagents that substantially optimize the reactions that need to occur during the assay and to further substantially optimize the sensitivity of the assay. Under appropriate circumstances, one or more of the reagents in the kit can be provided as a dry powder, usually lyophilized, including excipients, which upon dissolution will provide a reagent solution having the appropriate concentrations for performing a method or assay in accordance with the present disclosure. The kit can further include a written description of a method in accordance with any of the methods as described herein.

In one embodiment, a kit for the detection and/or quantification of one or more target molecules that may be present in a test sample includes at least one aptamer having specific affinity for a target molecule and comprising a tag; and a solid support, wherein the solid support includes at least one capture agent disposed thereon, and wherein the capture element is capable of associating with the tag on the aptamer.

In another embodiment, a kit for the detection and/or quantification of one or more target molecules that may be present in a test sample includes at least one aptamer having specific affinity for a target molecule and comprising a tag and a label; and a solid support, wherein the solid support includes at least one capture agent disposed thereon, and wherein the capture element is capable of associating with the tag on the aptamer.

In another embodiment, a kit for the detection and/or quantification of one or more target molecules that may be present in a test sample includes at least one aptamer having specific affinity for a target molecule and comprising a releasable tag and a label; and a solid support, wherein the solid support includes at least one capture agent disposed thereon, and wherein the capture element is capable of associating with the tag on the aptamer.

In addition, any of the above- described kits may contain reagents and materials for the performance of a kinetic challenge during the detection method of the kit.

As used herein "Catch-1" refers to the partitioning of an aptamer-target affinity complex or aptamer-target covalent complex. The purpose of Catch-1 is to remove substantially all of the components in the test sample that are not associated with the aptamer. Removing the majority of such components will generally improve target tagging efficiency by removing non-target molecules from the target tagging step used for Catch-2 capture and may lead to lower assay background. In one embodiment, a tag is attached to the aptamer either before the assay, during preparation of the assay, or during the assay by appending the tag to the aptamer. In one embodiment, the tag is a releasable tag. In one embodiment, the releasable tag comprises a cleavable linker and a tag. As described above, tagged aptamer can be captured on a solid support where the solid support comprises a capture element appropriate for the tag. The solid support can then be washed as described herein prior to equilibration with the test sample to remove any unwanted materials (Catch-0).

As used herein "Catch-2" refers to the partitioning of an aptamer-target affinity complex or aptamer-target covalent complex based on the capture of the target molecule. The purpose of the Catch-2 step is to remove free, or uncomplexed, aptamer from the test sample prior to detection and optional quantification. Removing free aptamer from the sample allows for the detection of the aptamer-target affinity or aptamer-target covalent complexes by any suitable nucleic acid detection technique. When using Q-PCR for detection and optional quantification, the removal of free aptamer is needed for accurate detection and quantification of the target molecule.

In one embodiment, the target molecule is a protein or peptide and free aptamer is partitioned from the aptamer-target affinity (or covalent) complex (and the rest of the test sample) using reagents that can be incorporated into proteins (and peptides) and complexes that include proteins (or peptides), such as, for example, an aptamer-target affinity (or covalent) complex. The tagged protein (or peptide) and aptamer-target affinity (or covalent) complex can be immobilized on a solid support, enabling partitioning of the protein (or peptide) and the aptamer-target affinity (or covalent) complex from free aptamer. Such tagging can include, for example, a biotin moiety that can be incorporated into the protein or peptide.

In one embodiment, a Catch-2 tag is attached to the protein (or peptide) either before the assay, during preparation of the assay, or during the assay by chemically attaching the tag to the targets. In one embodiment the Catch-2 tag is a releasable tag. In one embodiment, the releasable tag comprises a cleavable linker and a tag. It is generally not necessary, however, to release the protein (or peptide) from the Catch-2 solid support. As described above, tagged targets can be captured on a second solid support where the solid support comprises a capture element appropriate for the target tag. The solid support is then washed with various buffered solutions including buffered solutions comprising organic solvents and buffered solutions comprising salts and/or detergents containing salts and/or detergents.

After washing the second solid support, the aptamer-target affinity complexes are then subject to a dissociation step in which the complexes are disrupted to yield free aptamer while the target molecules generally remain bound to the solid support through the binding interaction of the capture element and target capture tag. The aptamer can be released from the aptamer-target affinity complex by any method that disrupts the structure of either the aptamer or the target. This may be achieved though washing of the support bound aptamer-target affinity complexes in high salt buffer which dissociates the non-covalently bound aptamer-target complexes. Eluted free aptamers are collected and detected. In another embodiment, high or low pH is used to disrupt the aptamer-target affinity complexes. In another embodiment high temperature is used to dissociate aptamer-target affinity complexes. In another embodiment, a combination of any of the above methods may be used. In another embodiment, proteolytic digestion of the protein moiety of the aptamer-target affinity complex is used to release the aptamer component.

In the case of aptamer-target covalent complexes, release of the aptamer for subsequent quantification is accomplished using a cleavable linker in the aptamer construct. In another embodiment, a cleavable linker in the target tag will result in the release of the aptamer-target covalent complex.

As used herein, "competitor molecule" and "competitor" are used interchangeably to refer to any molecule that can form a non-specific complex with a non-target molecule, for example to prevent that non-target molecule from rebinding non-specifically to an aptamer. "Competitor molecules" or "competitors" refer to more than one such set of molecules. Competitor molecules include oligonucleotides, polyanions (e.g., heparin, herring sperm DNA, single-stranded salmon sperm DNA, and polydextrans (e.g., dextran sulfate)), abasic phosphodiester polymers, dNTPs, and pyrophosphate. In the case of a kinetic challenge that uses a competitor, the competitor can also be any molecule that can form a non-specific complex with a free aptamer or protein, for example to prevent that aptamer or protein from rebinding non-specifically to a non-target molecule. Such competitor molecules include polycations (e.g., spermine, spermidine, polylysine, and polyarginine) and amino acids (e.g., arginine and lysine). When a competitor is used as the kinetic challenge a fairly high concentration is utilized relative to the anticipated concentration of total protein or total aptamer present in the sample. In one embodiment, about 10 mM dextran sulfate is used as the competitor in a kinetic challenge. In one embodiment, the kinetic challenge comprises adding a competitor to the mixture containing the aptamer-target affinity complex, and incubating the mixture containing the aptamer-target affinity complex for a time of greater than or equal to about 30 seconds, about 1 minute, about 2 minutes, about 3 minutes, about 4 minutes, about 5 minutes, about 10 minutes, about 30 minutes, and about 60 minutes. In another embodiment, the kinetic challenge comprises adding a competitor to the mixture containing the aptamer-target affinity complex and incubating the mixture containing the aptamer-target affinity complex for a time such that the ratio of the measured level of aptamer-target affinity complex to the measured level of the non-specific complex is increased.

In some embodiments, the kinetic challenge is performed by diluting the test sample with binding buffer or any other solution that does not significantly increase the natural rate of dissociation of aptamer-target affinity complexes. The dilution can be about 2X, about 3X, about 4X, about 5X, or any suitable greater dilution. Larger dilutions provide a more effective kinetic challenge by reducing the concentration of total protein and aptamer after dilution and, therefore, the rate of their re-association. If dilution is used to introduce a kinetic challenge, the subsequent test sample mixture containing the aptamer-target affinity complex may be concentrated before further processing. If applicable, this concentration can be accomplished using methods described herein with respect to the optional partitioning of any free aptamers from the test sample and/or the optional removal of other components of the test sample that can react with the tagging agent. When dilution is used as the kinetic challenge, the amount of dilution is selected to be as high as practical, in view of both the initial test sample volume and the desirability of recovering the aptamer-target affinity complex from the final (diluted) volume without incurring a significant loss of the complex. In one embodiment, the aptamer-target affinity complex is diluted and the mixture is incubated for a time ≥ about 30 seconds, ≥ about 1 minute, ≥ about 2 minutes, ≥ about 3 minutes, ≥ about 4 minutes, ≥ about 5 minutes, ≥ about 10 minutes, ≥ about 30 minutes, and ≥ about 60 minutes. In another embodiment, the aptamer-target affinity complex is diluted and the mixtures containing the aptamer-target affinity complex are incubated for a time such that the ratio of the measured level of aptamer-target affinity complex to the measured level of the non-specific complex is increased.

In some embodiments, the kinetic challenge is performed in such a manner that the effect of sample dilution and the effect of introducing a competitor are realized simultaneously. For example, a test sample can be diluted with a large volume of competitor. Combining these two kinetic challenge strategies may provide a more effective kinetic challenge than can be achieved using one strategy. In one embodiment, the dilution can be about 2X, about 3X, about 4X, about 5X, or any suitable greater dilution and the competitor is about 10 mM dextran sulfate. In one embodiment, the kinetic challenge comprises diluting the mixture containing the aptamer-target affinity complex, adding a competitor to the mixture containing the aptamer-target affinity complex, and incubating the mixture containing the aptamer-target affinity complex for a time greater than or equal to about 30 seconds, about 1 minute, about 2 minutes, about 3 minutes, about 4 minutes, about 5 minutes, about 10 minutes, about 30 minutes, and about 60 minutes. In another embodiment, the kinetic challenge comprises diluting the mixture containing the aptamer-target affinity complex, adding a competitor to the mixture containing the aptamer-target affinity complex and incubating the mixture containing the aptamer-target affinity complex for a time such that the ratio of the measured level of aptamer-target affinity complex to the measured level of the non-specific complex is increased.

As disclosed herein, an aptamer can further comprise a "tag," which refers to a component that provides a means for attaching or immobilizing an aptamer (and any target molecule that is bound to it) to a solid support. A "tag" is a moiety that is capable of associating with a "capture element". "Tags" or "capture elements" refers to more than one such set of components. The tag can be attached to or included in the aptamer by any suitable method. Generally, the tag allows the aptamer to associate, either directly or indirectly, with a capture element or receptor that is attached to the solid support. The capture element is typically chosen (or designed) to be highly specific in its interaction with the tag and to retain that association during subsequent processing steps or procedures. A tag can enable the localization of an aptamer-target affinity complex (or covalent aptamer-target affinity complex) to a spatially defined address on a solid support. Different tags, therefore, can enable the localization of different aptamer-target covalent complexes to different spatially defined addresses on a solid support. A tag can be a polynucleotide, a polypeptide, a peptide nucleic acid, a locked nucleic acid, an oligosaccharide, a polysaccharide, an antibody, an affibody, an antibody mimic, a cell receptor, a ligand, a lipid, biotin, polyhistidine, or any fragment or derivative of these structures, any combination of the foregoing, or any other structure with which a capture element (or linker molecule, as described below) can be designed or configured to bind or otherwise associate with specificity. Generally, a tag is configured such that it does not interact intramolecularly with either itself or the aptamer to which it is attached or of which it is a part. If SELEX is used to identify an aptamer, the tag may be added to the aptamer either pre- or post-SELEX. In one embodiment, the tag is included on the 5'-end of the aptamer post-SELEX. In another embodiment, the tag is included on the 3'-end of the aptamer post-SELEX. In yet another embodiment, tags may be included on both the 3' and 5' ends of the aptamers in a post-SELEX modification process. In another embodiment, the tag may be an internal segment of the aptamer.

In one embodiment, the tag is a biotin group and the capture element is a biotin binding protein such as avidin, streptavidin, neutravidin, Extravidin, or Traptavidin. This combination may be conveniently used in various embodiments, as biotin is easily incorporated into aptamers during synthesis and streptavidin beads are readily available.

In one embodiment, the tag is polyhistidine and the capture element is nitrilotriacetic acid (NTA) chelated with a metal ion such as nickel, cobalt, iron, or any other metal ion able to form a coordination compound with poly-histidine when chelated with NTA.

In one embodiment, the tag is a polynucleotide that is designed to hybridize directly with a capture element that contains a complementary polynucleotide sequence. In this case, the tag is sometimes referred to as a "sequence tag" and the capture element is generally referred to as a "probe". In this embodiment, the tag is generally configured and the hybridization reaction is carried out under conditions such that the tag does not hybridize with a probe other than the probe for which the tag is a perfect complement. This allows for the design of a multiplex assay format as each tag/probe combination can have unique sequences.

In some embodiments, the tag comprises nucleotides that are a part of the aptamer itself. For example, if SELEX is used to identify an aptamer, the aptamer generally includes a 5'-fixed end separated from a 3'-fixed end by a nucleotide sequence that varies, depending upon the aptamer, that is, a variable region. In one embodiment, the tag can comprise any suitable number of nucleotides included in a fixed end of the aptamer, such as, for example, an entire fixed end or any portion of a fixed end, including nucleotides that are internal to a fixed end. In another embodiment, the tag can comprise any suitable number of nucleotides included within the variable region of the aptamer, such as, for example, the entire variable region or any portion of the variable region. In a further embodiment, the tag can comprise any suitable number of nucleotides that overlap both the variable region and one of the fixed ends, that is, the tag can comprise a nucleotide sequence that includes any portion (including all) of the variable region and any portion (including all) of a fixed end.

In another embodiment, a tag can associate directly with a probe and covalently bind to the probe, thereby covalently linking the aptamer to the surface of the solid support. In this embodiment, the tag and the probe can include suitable reactive groups that, upon association of the tag with the probe, are sufficiently proximate to each other to undergo a chemical reaction that produces a covalent bond. The reaction may occur spontaneously or may require activation, such as, for example, photo-activation or chemical activation. In one embodiment, the tag includes a diene moiety and the probe includes a dienophile, and covalent bond formation results from a spontaneous Diels-Alder conjugation reaction of the diene and dienophile. Any appropriate complementary chemistry can be used, such as, for example, N-Mannich reaction, disulfide formation, Curtius reaction, Aldol condensation, Schiff base formation, and Michael addition.

In another embodiment, the tag associates indirectly with a probe, such as, for example, through a linker molecule, as further described below. In this embodiment, the tag can include a polynucleotide sequence that is complementary to a particular region or component of a linker molecule. The tag is generally configured and the hybridization reaction is carried out such that the tag does not hybridize with a polynucleotide sequence other than the polynucleotide sequence included in the linker molecule.

If the tag includes a polynucleotide, the polynucleotide can include any suitable number of nucleotides. In one embodiment, a tag includes at least about 10 nucleotides. In another embodiment, the tag includes from about 10 to about 45 nucleotides. In yet another embodiment, the tag includes at least about 30 nucleotides. Different tags that include a polynucleotide can include either the same number of nucleotides or a different number of nucleotides.

In some embodiments, the tag component is bi-functional in that it includes functionality for specific interaction with a capture element on a solid support or "probe" as defined below (probe association component), and functionality for dissociating the molecule to which it is attached from the probe association component of the tag. The means for dissociating the probe association component of the tag includes chemical means, photochemical means or other means depending upon the particular tag that is employed.

As used herein, "capture element", "probe" or "receptor" refers to a molecule that is configured to associate, either directly or indirectly, with a tag. A "capture element", "probe" or "receptor" is a set of copies of one type of molecule or one type of multimolecular structure that is capable of immobilizing the moiety to which the tag is attached to a solid support by associating, either directly or indirectly, with the tag. "Capture elements" "probes" or "receptors" refer to more than one such set of molecules. A capture element, probe or receptor can be a polynucleotide, a polypeptide, a peptide nucleic acid, a locked nucleic acid, an oligosaccharide, a polysaccharide, an antibody, an affibody, an antibody mimic, a cell receptor, a ligand, a lipid, biotin, polyhistidine, or any fragment or derivative of these structures, any combination of the foregoing, or any other structure with which a tag (or linker molecule) can be designed or configured to bind or otherwise associate with specificity. A capture element, probe or receptor can be attached to a solid support either covalently or non-covalently by any suitable method.

While the terms "capture element", "probe" and "receptor" are used interchangeably, probe generally refers to a polynucleotide sequence. In one embodiment, the probe includes a polynucleotide that has a sequence that is complementary to a polynucleotide tag sequence. In this embodiment, the probe sequence is generally configured and the hybridization reaction is carried out under conditions such that the probe does not hybridize with a nucleotide sequence other than the tag for which the probe includes the complementary sequence (*i.e.,* the probe is generally configured and the hybridization reaction is carried out under conditions such that the probe does not hybridize with a different tag or an aptamer).

In another embodiment, the probe associates indirectly with a tag, for example, through a linker molecule. In this embodiment, the probe can include a polynucleotide sequence that is complementary to a particular region or component of a linker molecule. The probe is generally configured and the hybridization reaction is carried out such that the probe does not hybridize with a polynucleotide sequence other than the polynucleotide sequence included in the linker molecule.

If a probe includes a polynucleotide, the polynucleotide can include any suitable number of nucleotides. In one embodiment, a probe includes at least about 10 nucleotides. In another embodiment, a probe includes from about 10 to about 45 nucleotides. In yet another embodiment, a probe includes at least about 30 nucleotides. Different probes that include a polynucleotide can include either the same number of nucleotides or a different number of nucleotides.

In some embodiments, the capture probe is bi-functional in that it includes functionality for specific interaction with a polynucleotide tag, and functionality for dissociating the probe from the solid support such that the probe and aptamer are simultaneously released. The means for dissociating the probe from the solid support includes chemical means, photochemical means or other means depending upon the particular capture probe that is employed.

Due to the reciprocal nature of the interaction between a particular tag and capture element pair, a tag in one embodiment may be used as a capture element in another embodiment, and a capture element in one embodiment may be used as a tag in another embodiment. For example, an aptamer with a biotin tag may be captured with streptavidin attached to a solid support in one embodiment, while an aptamer with a streptavidin tag may be captured with biotin attached to a solid support in another embodiment.

As used herein, a linker is a molecular structure that is use to connect two functional groups or molecular structures. As used herein, "spacing linker" or more simply a "spacer" refers to a group of benign atoms that provide separation or spacing between two different functional groups within an aptamer. As used herein, a "releasable" or "cleavable" element, moiety, or linker refers to a molecular structure that can be broken to produce two separate components. A releasable (or cleavable) element may comprise a single molecule in which a chemical bond can be broken (referred to herein as an "inline cleavable linker"), or it may comprise two or more molecules in which a non-covalent interaction can be broken or disrupted (referred to herein as a "hybridization linker").

In some embodiments, it is necessary to spatially separate certain functional groups from others in order to prevent interference with the individual functionalities. For example, the presence of a label, which absorbs certain wavelengths of light, proximate to a photocleavable group can interfere with the efficiency of photocleavage. It is therefore desirable to separate such groups with a non-interfering moiety that provides sufficient spatial separation to recover full activity of photocleavage, for example. In some embodiments, a "spacing linker" has been introduced into an aptamer with both a label and photocleavage functionality.

In one embodiment, spacing linkers are introduced into the aptamer during synthesis and so can be comprised of number of phosphoramidite spacers, including but limited to aliphatic carbon chains of length 3, 6, 9, 12 and 18 carbon atoms, polyethylene glycol chains of length 1, 3, and 9 ethylene glycol units, or a tetrahydrofuran moiety (termed dSpacer (Glenn Research) or any combination of the foregoing or any other structure or chemical component that can be designed or configured to add length along a phosphodiester backbone. In another embodiment, the spacing linker includes polynucleotides, such as poly dT, dA, dG, or dC or poly U, A, G, or C or any combination of the foregoing. In another embodiment, spacers include one or more abasic ribose or deoxyribose moieties. Note that such sequences are designed such that they do not interfere with the aptamer's structure or function.

As used herein, a "hybridization linker" refers to a linker that comprises two or more molecules in which a non-covalent interaction can be broken or disrupted through chemical or physical methods. In some embodiments, a hybridization linker is used to join an aptamer to a tag, thereby forming a releasable tag. For example, a hybridization linker can be utilized in any of the described assays to create a releasable connection between an aptamer and a biotin (e.g. in the affinity assays and crosslinking assays) or a releasable connection between an aptamer and a photocrosslinking group (e.g. in the crosslinking assays).

In one embodiment, a hybridization linker comprises two nucleic acids that hybridize to form a non-covalent bond. In one embodiment, one of the nucleic acids that forms the hybridization link can be a region of the aptamer itself and the other nucleic acid can be a nucleic acid that is complementary to that region. Release can be accomplished by any suitable mechanism for disrupting nucleic acid duplexes (while still maintaining compatibility with the assay). In one embodiment, 20 mM NaOH is used to disrupt the hybridization linker in the dual catch photocrosslinking assay. A hybridization linker molecule may have any suitable configuration and can include any suitable components, including one or more polynucleotides, polypeptides, peptide nucleic acids, locked nucleic acids, oligosaccharides, polysaccharides, antibodies, affibodies, antibody mimics or fragments, receptors, ligands, lipids, any fragment or derivative of these structures, any combination of the foregoing, or any other structure or chemical component that can be designed or configured to form a releasable structure.

In one embodiment, the releasable tag consists of at least one polynucleotide consisting of a suitable number of nucleotides. In one embodiment, a polynucleotide component of a linker molecule includes at least about 10 nucleotides. In another embodiment, a polynucleotide component of a linker molecule includes from about 10 to about 45 nucleotides. In yet another embodiment, a polynucleotide component of a linker molecule includes at least about 30 nucleotides. Linker molecules used in any of the methods disclosed herein can include polynucleotide components having either the same number of nucleotides or a different number of nucleotides.

"Solid support" refers to any substrate having a surface to which molecules may be attached, directly or indirectly, through either covalent or non-covalent bonds. The solid support may include any substrate material that is capable of providing physical support for the capture elements or probes that are attached to the surface. The material is generally capable of enduring conditions related to the attachment of the capture elements or probes to the surface and any subsequent treatment, handling, or processing encountered during the performance of an assay. The materials may be naturally occurring, synthetic, or a modification of a naturally occurring material. Suitable solid support materials may include silicon, a silicon wafer chip, graphite, mirrored surfaces, laminates, membranes, ceramics, plastics (including polymers such as, e.g., poly(vinyl chloride), cyclo-olefin copolymers, agarose gels or beads, polyacrylamide, polyacrylate, polyethylene, polypropylene, poly(4-methylbutene), polystyrene, polymethacrylate, poly(ethylene terephthalate), polytetrafluoroethylene (PTFE or Teflon^{®}), nylon, poly(vinyl butyrate)), germanium, gallium arsenide, gold, silver, Langmuir Blodgett films, a flow through chip, etc., either used by themselves or in conjunction with other materials. Additional rigid materials may be considered, such as glass, which includes silica and further includes, for example, glass that is available as Bioglass. Other materials that may be employed include porous materials, such as, for example, controlled pore glass beads, crosslinked beaded Sepharose^{®} or agarose resins, or copolymers of crosslinked bis-acrylamide and azalactone. Other beads include nanoparticles, polymer beads, solid core beads, paramagnetic beads, or microbeads. Any other materials known in the art that are capable of having one or more functional groups, such as any of an amino, carboxyl, thiol, or hydroxyl functional group, for example, incorporated on its surface, are also contemplated.

The material used for a solid support may take any of a variety of configurations ranging from simple to complex. The solid support can have any one of a number of shapes, including a strip, plate, disk, rod, particle, bead, tube, well (microtiter), and the like. The solid support may be porous or non-porous, magnetic, paramagnetic, or non-magnetic, polydisperse or monodisperse, hydrophilic or hydrophobic. The solid support may also be in the form of a gel or slurry of closely-packed (as in a column matrix) or loosely-packed particles.

In one embodiment, the solid support with attached capture element is used to capture tagged aptamer-target affinity complexes or aptamer-target covalent complexes from a test mixture. In one particular example, when the tag is a biotin moiety, the solid support could be a streptavidin-coated bead or resin such as Dynabeads M-280 Streptavidin, Dynabeads MyOne Streptavidin, Dynabeads M-270 Streptavidin (Invitrogen), Streptavidin Agarose Resin (Pierce), Streptavidin Ultralink Resin, MagnaBind Streptavidin Beads (ThermoFisher Scientific), BioMag Streptavidin, ProMag Streptavidin, Silica Streptavidin (Bangs Laboratories), Streptavidin Sepharose High Performance (GE Healthcare), Streptavidin Polystyrene Microspheres (Microspheres-Nanospheres), Streptavidin Coated Polystyrene Particles (Spherotech), or any other streptavidin coated bead or resin commonly used by one skilled in the art to capture biotin-tagged molecules.

As has been described above, one object of the instant invention is to convert a protein signal into an aptamer signal. As a result the quantity of aptamers collected/detected is indicative of, and may be directly proportional to, the quantity of target molecules bound and to the quantity of target molecules in the sample. A number of detection schemes can be employed without eluting the aptamer-target affinity or aptamer-target covalent complex from the second solid support after Catch-2 partitioning. In addition to the following embodiments of detection methods, other detection methods will be known to one skilled in the art.

Many detection methods require an explicit label to be incorporated into the aptamer prior to detection. In these embodiments, labels, such as, for example, fluorescent or chemiluminescent dyes can be incorporated into aptamers either during or post synthesis using standard techniques for nucleic acid synthesis. Radioactive labels can be incorporated either during synthesis or post synthesis using standard enzyme reactions with the appropriate reagents. Labeling can also occur after the Catch-2 partitioning and elution by using suitable enzymatic techniques. For example, using a primer with the above mentioned labels, PCR will incorporate labels into the amplification product of the eluted aptamers. When using a gel technique for quantification, different size mass labels can be incorporated using PCR as well. These mass labels can also incorporate different fluorescent or chemiluminescent dyes for additional multiplexing capacity. Labels may be added indirectly to aptamers by using a specific tag incorporated into the aptamer, either during synthesis or post synthetically, and then adding a probe that associates with the tag and carries the label. The labels include those described above as well as enzymes used in standard assays for colorimetric readouts, for example. These enzymes work in combination with enzyme substrates and include enzymes such as, for example, horseradish peroxidase (HRP) and alkaline phosphatase (AP). Labels may also include materials or compounds that are electrochemical functional groups for electrochemical detection.

For example, the aptamer may be labeled, as described above, with a radioactive isotope such as ³¹P prior to contacting the test sample. Employing any one of the four basic assays, and variations thereof as discussed above, aptamer detection may be simply accomplished by quantifying the radioactivity on the second solid support at the end of the assay. The counts of radioactivity will be directly proportional to the amount of target in the original test sample. Similarly, labeling an aptamer with a fluorescent dye, as described above, before contacting the test sample allows for a simple fluorescent readout directly on the second solid support. A chemiluminescent label or a quantum dot can be similarly employed for direct readout from the second solid support, requiring no aptamer elution.

By eluting the aptamer or releasing photoaptamer-target covalent complex from the second solid support additional detection schemes can be employed in addition to those described above. For example, the released aptamer, photoaptamer or photoaptamer-target covalent complex can be run on a PAGE gel and detected and optionally quantified with a nucleic acid stain, such as SYBR Gold. Alternatively, the released aptamer, photoaptamer or photoaptamer covalent complex can be detected and quantified using capillary gel electrophoresis (CGE) using a fluorescent label incorporated in the aptamer as described above. Another detection scheme employs quantitative PCR to detect and quantify the eluted aptamer using SYBR Green, for example. Alternatively, the Invader® DNA assay may be employed to detect and quantify the eluted aptamer. Another alternative detection scheme employs next generation sequencing.

In another embodiment, the amount or concentration of the aptamer-target affinity complex (or aptamer-target covalent complex) is determined using a "molecular beacon" during a replicative process (see, e.g., Tyagi et al., Nat. Biotech. 16:49 53, 1998; U.S. Pat. No. 5,925,517). A molecular beacon is a specific nucleic acid probe that folds into a hairpin loop and contains a fluorophore on one end and a quencher on the other end of the hairpin structure such that little or no signal is generated by the fluorophore when the hairpin is formed. The loop sequence is specific for a target polynucleotide sequence and, upon hybridizing to the aptamer sequence the hairpin unfolds and thereby generates a fluorescent signal.

For multiplexed detection of a small number of aptamers still bound to the second solid support, fluorescent dyes with different excitation/emission spectra can be employed to detect and quantify two, or three, or five, or up to ten individual aptamers. Similarly different sized quantum dots can be employed for multiplexed readouts. The quantum dots can be introduced after partitioning free aptamer from the second solid support. By using aptamer specific hybridization sequences attached to unique quantum dots multiplexed readings for 2, 3, 5, and up to 10 aptamers can be performed. Labeling different aptamers with different radioactive isotopes that can be individually detected, such as ³²P, ¹²⁵I, ³H, ¹³C, and ³⁵S, can also be used for limited multiplex readouts.

For multiplexed detection of aptamers released from the Catch-2 second solid support, a single fluorescent dye, incorporated into each aptamer as described above, can be used with a quantification method that allows for the identification of the aptamer sequence along with quantification of the aptamer level. Methods include but are not limited to DNA chip hybridization, micro-bead hybridization, next generation sequencing and CGE analysis.

In one embodiment, a standard DNA hybridization array, or chip, is used to hybridize each aptamer or photoaptamer to a unique or series of unique probes immobilized on a slide or chip such as Agilent arrays, Illumina BeadChip Arrays, NimbleGen arrays or custom printed arrays. Each unique probe is complementary to a sequence on the aptamer. The complementary sequence may be a unique hybridization tag incorporated in the aptamer, or a portion of the aptamer sequence, or the entire aptamer sequence. The aptamers released from the Catch-2 solid support are added to an appropriate hybridization buffer and processed using standard hybridization methods. For example, the aptamer solution is incubated for 12 hours with a DNA hybridization array at about 60°C to ensure stringency of hybridization. The arrays are washed and then scanned in a fluorescent slide scanner, producing an image of the aptamer hybridization intensity on each feature of the array. Image segmentation and quantification is accomplished using image processing software, such as ArrayVision. In one embodiment, multiplexed aptamer assays can be detected using up to 25 aptamers, up to 50 aptamers, up to 100 aptamers, up to 200 aptamers, up to 500 aptamers, up to 1000 aptamers, and up to 10,000 aptamers.

In one embodiment, addressable micro-beads having unique DNA probes complementary to the aptamers as described above are used for hybridization. The micro-beads may be addressable with unique fluorescent dyes, such as Luminex beads technology, or use bar code labels as in the Illumina VeraCode technology, or laser powered transponders. In one embodiment, the aptamers released from the Catch-2 solid support are added to an appropriate hybridization buffer and processed using standard micro-bead hybridization methods. For example, the aptamer solution is incubated for two hours with a set of micro-beads at about 60°C to ensure stringency of hybridization. The solutions are then processed on a Luminex instrument which counts the individual bead types and quantifies the aptamer fluorescent signal. In another embodiment, the VeraCode beads are contacted with the aptamer solution and hybridized for two hours at about 60°C and then deposited on a gridded surface and scanned using a slide scanner for identification and fluorescence quantification. In another embodiment, the transponder micro-beads are incubated with the aptamer sample at about 60°C and then quantified using an appropriate device for the transponder micro-beads. In one embodiment, multiplex aptamer assays can be detected by hybridization to micro-beads using up to 25 aptamers, up to 50 aptamers, up to 100 aptamers, up to 200 aptamers, and up to 500 aptamers.

The sample containing the eluted aptamers can be processed to incorporate unique mass tags along with fluorescent labels as described above. The mass labeled aptamers are then injected into a CGE instrument, essentially a DNA sequencer, and the aptamers are identified by their unique masses and quantified using fluorescence from the dye incorporated during the labeling reaction. One exemplary example of this technique has been developed by Althea Technologies.

In many of the methods described above, the solution of aptamers can be amplified and optionally tagged before quantification. Standard PCR amplification can be used with the solution of aptamers eluted from the Catch-2 solid support. Such amplification can be used prior to DNA array hybridization, micro-bead hybridization, and CGE readout.

In another embodiment, the aptamer-target affinity complex (or aptamer-target covalent complex) is detected and/or quantified using Q-PCR. As used herein, "Q-PCR" refers to a PCR reaction performed in such a way and under such controlled conditions that the results of the assay are quantitative, that is, the assay is capable of quantifying the amount or concentration of aptamer present in the test sample.

In one embodiment, the amount or concentration of the aptamer-target affinity complex (or aptamer-target covalent complex) in the test sample is determined using TaqMan^{®} PCR. This technique generally relies on the 5'-3' exonuclease activity of the oligonucleotide replicating enzyme to generate a signal from a targeted sequence. A TaqMan probe is selected based upon the sequence of the aptamer to be quantified and generally includes a 5'-end fluorophore, such as 6-carboxyfluorescein, for example, and a 3'-end quencher, such as, for example, a 6-carboxytetramethylfluorescein, to generate signal as the aptamer sequence is amplified using polymerase chain reaction (PCR). As the polymerase copies the aptamer sequence, the exonuclease activity frees the fluorophore from the probe, which is annealed downstream from the PCR primers, thereby generating signal. The signal increases as replicative product is produced. The amount of PCR product depends upon both the number of replicative cycles performed as well as the starting concentration of the aptamer.

In another embodiment, the amount or concentration of an aptamer-target affinity complex (or aptamer-target covalent complex) is determined using an intercalating fluorescent dye during the replicative process. The intercalating dye, such as, for example, SYBR^{®} green, generates a large fluorescent signal in the presence of double-stranded DNA as compared to the fluorescent signal generated in the presence of single-stranded DNA. As the double-stranded DNA product is formed during PCR, the signal produced by the dye increases. The magnitude of the signal produced is dependent upon both the number of PCR cycles and the starting concentration of the aptamer.

In another embodiment, the aptamer-target affinity complex (or aptamer-target covalent complex) is detected and/or quantified using mass spectrometry. Unique mass tags can be introduced using enzymatic techniques described above. For mass spectroscopy readout, no detection label is required, rather the mass itself is used to both identify and, using techniques commonly used by those skilled in the art, quantified based on the location and area under the mass peaks generated during the mass spectroscopy analysis. An example using mass spectroscopy is the MassARRAY^{®} system developed by Sequenom.

A computer program may be utilized to carry out one or more steps of any of the methods disclosed herein. Another aspect of the present disclosure is a computer program product comprising a computer readable storage medium having a computer program stored thereon which, when loaded into a computer, performs or assists in the performance of any of the methods disclosed herein.

One aspect of the disclosure is a product of any of the methods disclosed herein, namely, an assay result, which may be evaluated at the site of the testing or it may be shipped to another site for evaluation and communication to an interested party at a remote location, if desired. As used herein, "remote location" refers to a location that is physically different than that at which the results are obtained. Accordingly, the results may be sent to a different room, a different building, a different part of city, a different city, and so forth. The data may be transmitted by any suitable means such as, e.g., facsimile, mail, overnight delivery, e-mail, ftp, voice mail, and the like.

"Communicating" information refers to the transmission of the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

### EXAMPLES

The following examples are provided for illustrative purposes only and are not intended to limit the scope of the invention as defined in the appended claims.

The foregoing describes the disclosure with reference to various embodiments and examples. No particular embodiment, example, or element of a particular embodiment or example is to be construed as a critical, required, or essential element or feature of any of the claims.

It will be appreciated that various modifications and substitutions can be made to the disclosed embodiments without departing from the scope of the disclosure as set forth in the claims below. The specification, including the figures and examples, is to be regarded in an illustrative manner, rather than a restrictive one, and all such modifications and substitutions are intended to be included within the scope of the disclosure. Accordingly, the scope of the disclosure may be determined by the appended claims and their legal equivalents, rather than by the examples. For example, steps recited in any of the method or process claims may be executed in any feasible order and are not limited to an order presented in any of the embodiments, the examples, or the claims.

### Proteomic Affinity Assay

### Catch-0

133 µL 7.5% streptavidin-agarose slurry in 1xSB17,Tw (40 mM HEPES, 102 mM NaCl, 1 mM EDTA, 5 mM MgCl₂, 5 mM KCl, 0.05% Tween-20) was added to wells of the filter plate (0.45 µm Millipore HV plates (Durapore cat# MAHVN4550)). The appropriate 1.1x aptamer mix (all aptamers contain a Cy3 fluorophore and a photo-cleavable biotin moiety on the 5' end) was thawed followed by vortexing. The 1.1x aptamer mix was then boiled for 10 min, vortexed for 30 s and allowed to cool to 20°C in a water bath for 20 min. The liquid in the filter plates containing the streptavidin agarose slurry was then removed by centrifugation (1000x g for 1 minute). 100 µL aptamer mix was added to the wells of the filter plate (robotically). The mixture was incubated at 25°C for 20 min on a shaker set at 850 rpm, protected from light.

### Catch-0 washes

Subsequent to the 20 min incubation the solution was removed via vacuum filtration. 190 µL 1x CAPS aptamer prewash buffer (50 mM CAPS, 1 mM EDTA, 0.05% Tw-20, pH 11.0) was added and the mixture was incubated for 1 minutes while shaking. The CAPS wash solution was then removed via vacuum filtration. The CAPS wash was then repeated one time. 190 µL 1xSX17,Tw was added and the mixture was incubated for 1 min while shaking. The 1xSB17,Tw was then removed via vacuum filtration. An additional 190 µL 1xSX17,Tw was added and the mixture was incubated for 1 min while shaking. The 1xSB17,Tw was then removed by centrifugation (1 min at 1000x g). Following removal of the 1xSB17,Tw, 150 µL Catch-0 storage buffer (150 mM NaCl, 40 mM HEPES, 1 mM EDTA, 0.02% sodium azide, 0.05% Tween-20) was added and the filter plate was carefully sealed at the plate perimeter only and stored at 4°C in the dark until use.

### Sample Preparation

Seventy-five µL of 40% sample diluent were plated out in a 40% sample plate (Final 40% sample contains: 20 µM Z-block, 1 mM benzamidine, 1 mM EGTA, 40 mM HEPES, 5 mM MgCl₂, 5 mM KCl, 1% Tween-20). One hundred ninety-five µL 1xSB17,Tw were plated out in a 1% sample plate. Ninety µL 1xSB17,Tw were plated out in a 1 to 10 dilution plate. One hundred thirty-three µL 1xSB17,Tw were plated out in a 0.005% sample plate. Samples were thawed for 10 min on the Rack Thawing Station in a 25°C incubator, then vortexed and spun at 1000x g for 1 minute. The caps were removed from the tubes. The samples were mixed (5 times with 50 µL) and 50 µL 100% sample was transferred to the 40% sample plate containing the sample diluents. The 40% sample was then mixed on the sample plate by pipetting up and down (110 µL 10 times). Five µL of 40% sample was then transferred to the 1% sample plate containing 1xSB17,Tw. Again this sample was mixed by pipetting up and down (120 µL 10 times). After mixing, 10 µL of the 1% sample was transferred to the 1 to 10 dilution plate containing 1xSB17,Tw, which was mixed by pipetting up and down (75 µL 10 times). Seven µL of the 0.1% sample from the 1 to 10 dilution plate was transferred into the 0.005% sample plate containing 1xSB17,Tw and mixed by pipetting up and down (110 µL 10 times).

### Plate Preparation before Equilibration

The Catch-0 storage solution was removed from the filter plates via vacuum filtration. One hundred ninety µL 1xSB17,Tw was then added followed by removal from the filter plates via vacuum filtration. An additional 190 µL 1xSB17,Tw was then added to the filter plates.

### Equilibration

The 1xSB17,Tw buffer was removed from the filter plates by centrifugation (1 min at 1000x g). 100 µL of the appropriate sample dilution was added to the filter plates (three filter plates, one for each sample dilution 40%, 1%, or 0.005%). The filter plates were carefully sealed at the plate perimeter only, avoiding pressurizing the wells. Pressure will cause leakage during equilibration. The plates were then incubated for 3.5 hours at 28°C on the thermoshaker set at 850 rpm, protected from light.

### Filter Plate Processing

After equilibration the filter plates were placed onto vacuum manifolds and the sample was removed by vacuum filtration. One hundred ninety µL biotin wash (100 µM biotin in 1xSB17,Tw) was added and the liquid was removed by vacuum filtration. The sample was then washed 5x with 190 µL 1xSB17,Tw (vacuum filtration). One hundred µL of 1 mM NHS-biotin in 1xSB17,Tw (freshly prepared) was added and the filter plates were blotted on an absorbent pad and the mixture was incubated for 5 minutes with shaking. The liquid was removed by vacuum filtration. One hundred and twenty five µL 20 mM glycine in 1xSB17,Tw was added and the liquid was removed by vacuum filtration. Again 125 µL 20 mM glycine in 1xSB17,Tw was added and the liquid removed by vacuum filtration. Subsequently the samples were washed 6x with 190 µL 1xSB17,Tw, with the liquid being removed by vacuum filtration. Eighty five µL photocleavage buffer (2 µM Z-block in 1xSB17,Tw) was then added to each of the filter plates.

### Photocleavage

The filter plates were blotted on absorbent pads and were irradiated for 6 min with a BlackRay UV lamp with shaking (800 rpm, 25°C). The plates were rotated 180 degrees and irradiated for an additional 6 min under the BlackRay light source. The 40% filter plate was placed onto an empty 96-well plate. The 1% filter plate was stacked on top of the 40% filter plate and the 0.005% filter plate was stacked on top of the 1% filter plate. The assembly of plated were spun for 1 min at 1000x g. The 96-well plate with eluted sample was placed onto the robot deck. 60% glycerol in 1xSB17,Tw from the 37°C incubator was placed onto the robotic deck.

### Catch-2

During assay setup 50 µL of 10 mg/mL MyOne SA beads (500 µg) was added to an ABgene Omni-tube 96-well plate for Catch-2 and placed in the Cytomat. The Catch-2 96-well bead plate was suspended for 90 s, placed on magnet block for 60 s and the supernatant was removed. All Catch-1 eluate was transferred to the Catch-2 bead plate and incubated on a Peltier thermoshaker (1350 rpm, 5 min, 25°C). The plate was transferred to a 25°C magnet for 2 minutes and the supernatant was removed. Next 75 µL 1xSB17,Tw was added and the sample and incubated on a Peltier shaker at 1350 rpm for 1 minute at 37°C. Then75 µL 60% glycerol in 1xSB17,Tw (heated to 37°C) was added and the sample was again incubated on the Peltier Shaker at 1350 rpm for 1 minute at 37°C. The plate was transferred to a magnet heated to 37°C and incubated for 2 min followed by the removal of the supernatant. This 37°C 1xSB17,Tw and glycerol wash cycle was repeated two more times. The sample was then washed to remove residual glycerol with 150 µL 1xSB17,Tw on a Peltier shaker (1350 rpm, 1 minute, 25°C), followed by 1 minute on a 25°C magnetic block. The supernatant was removed and 150 µL 1xSB17,Tw substituted with 0.5 M NaCl was added and incubated at 1350 rpm for 1 minute (25°C) followed by 1 minute on a 25°C magnetic block. The supernatant was removed and 75 µL perchlorate elution buffer (1.8 M NaClO₄, 40 mM PIPES, 1 mM EDTA, 0.05% Triton X-100, 1x Hybridization controls, pH=6.8) was added followed by a 10 minute incubation on a Peltier shaker (25°C, 1350 rpm). Afterwards the plate was transferred to a magnetic separator and incubated for 90 s, and the supernatant was recovered.

### Hybridization

Twenty µL eluted sample was added robotically to an empty the 96-well plate. Five µL 10x Agilent blocking buffer containing a second set of hybridization controls were robotically added to the eluted samples. Then 25 µL 2x Agilent HiRPM hybridization buffer was added manually to the wells. 40 µL of hybridization mix was loaded onto the Agilent gasket slide. The Agilent 8 by 15k array was added onto gasket slide and the sandwich was tightened with a clamp. The sandwich was then incubated rotating (20 rpm) for 19 hours at 55°C.

### Post-Hybridization Washing

Post hybridization slide processing was performed on a Little Dipper Processor (SciGene, Cat# 1080-40-1). Approximately 750 mL wash buffer 1 (Oligo aCGH/ChIP-on-chip Wash Buffer 1, Agilent Technologies) was placed into one glass staining dish. Approximately 750 mL wash buffer 1 (Oligo aCGH/ChIP-on-chip Wash Buffer 1, Agilent Technologies) was placed into Bath #1 of the Little Dipper Processor. Approximately 750 mL wash buffer 2 (Oligo aCGH/ChIP-on-chip Wash Buffer 1, Agilent Technologies) heated to 37°C was placed into Bath #2 of the Little Dipper Processor. The magnetic stir speed for both bath were set to 5. The temperature controller for Bath #1 was not turned on, while the temperature controller for Bath #2 was set to 37°C. Up to twelve slide/gasket assemblies were sequentially disassembled into the first staining dish containing Wash Buffer 1 and the slides were placed into a slide rack while still submerged in Wash Buffer 1. Once all slide/gaskets assemblies were disassembled, the slide rack was quickly transferred into Bath #1 of the Little Dipper Processor and the automated wash protocol was started. The Little Dipper Processor incubated the slides for 300 s in Bath #1 at a speed of 250 followed by a transfer to the 37°C Bath #2 containing the Agilent Wash 2 (Oligo aCGH/ChIP-on-chip Wash Buffer 2, Agilent Technologies) and incubated for 300 s at speed 100. Afterwards the Little Dipper Processor transferred the slide rack to the built-in centrifuge, where the slides were spun for 300 s at speed 690.

### Microarray Imaging

The microarray slides were imaged with a microarray scanner (Agilent G2565CA Microarray Scanner System, Agilent Technologies) in the Cy3-channel at 5 µm resolution at 100% PMT setting and the XRD option enabled at 0.05. The resulting tiff images were processed using Agilent feature extraction software version 10.7.3.1 with the GE1_107_Sep09 protocol.

## Claims

1. A method comprising:
providing an aptamer that is immobilized on a first solid support, the aptamer having a specific binding affinity for the target molecule and bearing a first tag having an affinity to a first capture element, the first solid support comprising a first capture element, and the first tag being associated with the first capture element to immobilize the aptamer on the first solid support,
said first solid support having been washed with one or more solutions that dissociate aggregated aptamers;
contacting said immobilized aptamer with the test sample, wherein an aptamer-target affinity complex is formed if said target molecule is present in said test sample;
removing one or more components of the mixture not associated with said first solid support;
attaching a second tag with an affinity to a second capture element to said target molecule in the aptamer-target affinity complex;
releasing the aptamer-target affinity complex from said first solid support;
exposing the released aptamer-target affinity complex to a second solid support comprising a second capture element and allowing the second tag to associate with said second capture element;
removing any components of the mixture not associated with said second solid support; and
eluting aptamers from said second solid support with one or more solutions comprising a chaotropic salt that disrupts aptamer/analyte interactions.

2. A method of detecting the presence of, or determining the amount of, a target molecule in a sample, the method comprising:
providing a plurality of immobilized aptamers, wherein each of said aptamers is specific to a target molecule, and wherein each of said plurality of aptamers comprises a first cleavable capture tag, said aptamers being immobilized on a solid support having probes adhered to the surface thereof, the probes binding to the first tag, such that the aptamers are immobilized onto the solid support through binding of the first tag and the probe;
contacting the immobilized aptamers with a sample containing target molecules to form a mixture containing aptamer-target molecule complexes bound to the solid support;
partitioning aptamer-target molecule complexes bound to the solid support from the remainder of the mixture;
introducing a second capture tag to the target molecule component of the aptamer-target molecule complexes;
dissociating the aptamer-target molecule complexes from the surface of the solid support by cleaving the first cleavable capture tag;
providing a second solid support having probes adhered to the surface of the support, wherein the probes are capable of binding to the second capture tag on target molecules;
contacting the dissociated aptamer-target molecule complexes with the second solid support such that the aptamer-target molecule complexes become bound to the second support through binding of the second capture tag and probe;
eluting aptamers from said second solid support with one or more buffered solutions comprising a chaotropic salt that disrupts aptamer/analyte interactions but supports aptamer/aptamer interactions and DNA hybridization;
dissociating the aptamer-target molecule;
detecting the free aptamers

3. The method of claim 1 or claim 2, wherein said aptamer comprises at least one C-5 modified nucleotide.

4. The method of claim 1, 2 or 3, wherein said aptamer comprises at least one chemical modification comprising a chemical substitution at one or more positions independently selected from a ribose position, a deoxyribose position, a phosphate position, and a base position, wherein optionally said chemical modification is independently selected from the group consisting of a 2'-position sugar modification, a 2'-amino (2'-NH₂), a 2'-fluoro (2'-F), a 2'-O-methyl (2'-OMe), a 5-position pyrimidine modification, an 8-position purine modification, a modification at a cytosine exocyclic amine, a substitution of 5-bromouracil, a substitution of 5-bromodeoxyuridine, a substitution of 5-bromodeoxycytidine, a backbone modification, methylation, a 3' cap, and a 5' cap.

5. The method of any one of claims 1 to 4, further comprising introducing a kinetic challenge.

6. The method of any one of claims 1 to 5, wherein said aptamer-target affinity complex has a slow rate of dissociation.

7. The method of claim 6, wherein the rate of dissociation of said aptamer-target affinity complex (t_{1/2}) is:
(a) greater than or equal to 30 minutes;
(b) between about 30 minutes and about 240 minutes; or
(c) selected from the group consisting of ≥ 30 minutes, ≥ 60 minutes, ≥ 90 minutes, ≥ 120 minutes, ≥ 150 minutes, ≥ 180 minutes, ≥ 210 minutes, and ≥ 240 minutes.

8. The method of claim 1, wherein one or more of said buffered solutions comprises an organic solvent, optionally glycerol.

9. The method of claim 1, wherein said chaotropic salt is selected from the group consisting of sodium perchlorate, lithium chloride, magnesium chloride and sodium chloride.

10. The method of any one of claims 1 to 9, wherein said aptamer is detected and optionally quantified using a method selected from the group consisting of Q-PCR, MS, next-generation sequencing and hybridization, wherein said Q-PCR is optionally performed using TaqMan^{®} PCR, an intercalating fluorescent dye during the PCR process, or a molecular beacon during the PCR process.

11. The method of any of claims 1 to 10, wherein the aptamer comprises a detectable moiety, wherein said detectable moiety is optionally selected from the group consisting of a dye, a quantum dot, a radiolabel, a electrochemical functional group, and an enzyme plus a detectable enzyme substrate, wherein said dye is preferably a fluorescent dye.

12. The method of any one of claims 1 to 11, wherein said aptamer is a single-stranded nucleic acid or a double-stranded nucleic acid.

13. The method of claim 12, wherein said aptamer comprises DNA, RNA or both DNA and RNA.

14. The method of any one of claims 1 to 13, wherein said target molecule is selected from the group consisting of a protein, a peptide, a carbohydrate, a polysaccharide, a glycoprotein, a hormone, a receptor, an antigen, an antibody, a virus, a substrate, a metabolite, a transition state analog, a cofactor, an inhibitor, a drug, a dye, a nutrient, a growth factor, a tissue, and a controlled substance, preferably wherein said target molecule is a protein or a peptide.

15. The method of any one of claims 1 to 14, wherein said test sample is selected from the group consisting of a biological sample, an environmental sample, a chemical sample, a pharmaceutical sample, a food sample, an agricultural sample, and a veterinary sample, wherein optionally the biological sample selected from the group consisting of blood, whole blood, leukocytes, peripheral blood mononuclear cells, plasma, serum, sputum, breath, urine, semen, saliva, meningeal fluid, amniotic fluid, glandular fluid, lymph fluid, nipple aspirate, bronchial aspirate, synovial fluid, joint aspirate, cells, a cellular extract, stool, tissue, a tissue extract, a tissue biopsy, and cerebrospinal fluid, preferably plasma or serum.

16. The method of any one of claims 1 to 15, wherein said first tag and said second tag, said first capture element and said second capture element each comprises at least one component independently selected from the group consisting of a polynucleotide, a polypeptide, a peptide nucleic acid, a locked nucleic acid, an oligosaccharide, a polysaccharide, an antibody, an affibody, an antibody mimic, a cell receptor, a ligand, a lipid, biotin, avidin, streptavidin, Extravidin, neutravidin, Traptavidin, a metal, histidine, and any portion of any of these structures.

17. The method of claim 1 or claim 2, wherein the first tag comprises a releasable moiety, preferably wherein the releasable moiety comprises a photocleavable moiety.

18. The method of claim 1 or claim 2, wherein said first solid support and second solid support each is independently selected from the group consisting of a polymer bead, an agarose bead, a polystyrene bead, an acrylamide bead, a solid core bead, a porous bead, a paramagnetic bead, glass bead, controlled pore bead, a microtitre well, a cyclo-olefin copolymer substrate, a membrane, a plastic substrate, nylon, a Langmuir-Blodgett film, glass, a germanium substrate, a silicon substrate, a silicon wafer chip, a flow through chip, a microbead, a nanoparticle, a polytetrafluoroethylene substrate, a polystyrene substrate, a gallium arsenide substrate, a gold substrate, and a silver substrate.

19. The method of claim 1 or claim 2, further comprising quantifying said target by quantifying said aptamer.

20. The method of claim 1 or claim 2, wherein detection of the aptamer comprises hybridizing the aptamer to a third solid support wherein the third solid support comprises a plurality of addressable features and wherein at least one of said features comprises at least capture element disposed thereon that is complementary to any sequence contained within the aptamer and/or further comprising the step of detecting said target molecule by detecting the aptamer portion of said aptamer-target affinity complex.

## Patentansprüche

1. Verfahren, umfassend:
Bereitstellen eines Aptamers, das auf einem ersten festen Träger immobilisiert ist, wobei das Aptamer eine spezifische Bindungsaffinität zu dem Zielmolekül aufweist und eine erste Markierung trägt, die eine Affinität zu einem ersten Fangelement aufweist, wobei der erste feste Träger ein erstes Fangelement umfasst und die erste Markierung mit dem ersten Fangelement assoziiert ist, um das Aptamer auf dem ersten festen Träger zu immobilisieren,
wobei der erste feste Träger mit einer oder mehreren Lösungen gewaschen wurde, die aggregierte Aptamere dissoziieren;
Kontaktieren des immobilisierten Aptamers mit der Testprobe, wobei ein Aptamer-Ziel-Affinitätskomplex ausgebildet wird, wenn das Zielmolekül in der Testprobe vorhanden ist;
Entfernen einer oder mehrerer Komponenten des Gemischs, die nicht mit dem ersten festen Träger assoziiert sind;
Binden einer zweiten Markierung mit einer Affinität zu einem zweiten Fangelement an das Zielmolekül in dem Aptamer-Ziel-Affinitätskomplex;
Freisetzen des Aptamer-Ziel-Affinitätskomplexes von dem ersten festen Träger;
Aussetzen des freigesetzten Aptamer-Ziel-Affinitätskomplexes gegenüber einem zweiten festen Träger, der ein zweites Fangelement umfasst, und das Zulassen der Assoziierung der zweiten Markierung mit dem zweiten Fangelement;
Entfernen jeglicher Komponenten des Gemischs, die nicht mit dem zweiten festen Träger assoziiert sind; und
Eluieren von Aptameren von dem zweiten festen Träger mit einer oder mehreren Lösungen, die ein chaotropes Salz umfassen, das Aptamer/Analyt-Wechselwirkungen unterbricht.

2. Verfahren zum Detektieren der Gegenwart von oder Bestimmen der Menge eines Zielmoleküls in einer Probe, wobei das Verfahren Folgendes umfasst:
Bereitstellen einer Vielzahl von immobilisierten Aptameren, wobei jedes der Aptamere spezifisch für ein Zielmolekül ist und wobei jedes der Vielzahl von Aptameren eine erste abspaltbare Fangmarkierung umfasst, wobei die Aptamere auf einem festen Träger immobilisiert werden, auf dessen Oberfläche Sonden haften, wobei die Sonden an die erste Markierung binden, sodass die Aptamere auf dem festen Träger durch Binden der ersten Markierung und der Sonde immobilisiert werden;
Kontaktieren der immobilisierten Aptamere mit einer Zielmoleküle enthaltenden Probe, um ein Gemisch zu bilden, das an den festen Träger gebundene Aptamer-Zielmolekül-Komplexe enthält;
Trennen der an den festen Träger gebundenen Aptamer-Zielmolekül-Komplexe von dem Rest des Gemischs;
Einführen einer zweiten Fangmarkierung zu der Zielmolekülkomponente des Aptamer-Zielmolekül-Komplexes;
Dissoziieren des Aptamer-Zielmolekül-Komplexes von der Oberfläche des festen Trägers durch Abspalten der ersten abspaltbaren Fangmarkierung;
Bereitstellen eines zweiten festen Trägers, an dessen Oberfläche Sonden haften, wobei die Sonden in der Lage sind, an die zweite Fangmarkierung auf Zielmolekülen zu binden;
Kontaktieren des dissoziierten Aptamer-Zielmolekül-Komplexes mit dem zweiten festen Träger, sodass die Aptamer-Zielmolekül-Komplexe durch Binden der zweiten Fangmarkierung und der Sonde an den zweiten Träger gebunden werden;
Eluieren von Aptameren von dem zweiten festen Träger mit einer oder mehreren Pufferlösungen, die ein chaotropes Salz umfassen, das Aptamer/Analyt-Wechselwirkungen unterbricht, aber Aptamer/Aptamer-Wechselwirkungen und DNA-Hybridisierung unterstützt;
Dissoziieren des Aptamer-Zielmoleküls;
Detektieren der freien Aptamere.

3. Verfahren nach Anspruch 1 oder 2, wobei das Aptamer zumindest ein C-5-modifiziertes Nucleotid umfasst.

4. Verfahren nach Anspruch 1, 2 oder 3, wobei das Aptamer zumindest eine chemische Modifikation umfasst, die eine chemische Substitution an einer oder mehreren Positionen umfasst, die unabhängig voneinander aus einer Riboseposition, einer Desoxyriboseposition, einer Phosphatposition und einer Basenposition ausgewählt sind, wobei die chemische Modifikation gegebenenfalls unabhängig aus der aus einer 2'-Position-Zuckermodifikation, einer 2'-Amino- (2'-NH₂-), einer 2'-Fluor- (2'-F-), einer 2'-O-Methyl- (2'-OMe-), einer 5-Position-Pyrimidin-Modifikation, einer 8-Position-Purin-Modifikation, einer Modifikation an einem exozyklischen Cytosinamin, einer Substitution von 5-Bromuracil, einer Substitution von 5-Bromdesoxyuridin, einer Substitution von 5-Bromdesoxycytidin, einer Hauptkettenmodifikation, Methylierung, einer 3'-Verkappung und einer 5'-Verkappung bestehenden Gruppe ausgewählt ist.

5. Verfahren nach einem der Ansprüche 1 bis 4, weiters umfassend das Einführen einer kinetischen Exposition.

6. Verfahren nach einem der Ansprüche 1 bis 5, wobei der Aptamer-Ziel-Affinitätskomplex eine langsame Dissoziationsrate aufweist.

7. Verfahren nach Anspruch 6, wobei die Dissoziationsrate des Aptamer-Ziel-Affinitätskomplexes (t_{1/2}):
(a) größer oder gleich 30 Minuten;
(b) zwischen etwa 30 Minuten und etwa 240 Minuten; oder
(c) aus der aus ≥ 30 Minuten, ≥ 60 Minuten, ≥ 90 Minuten, ≥ 120 Minuten, ≥ 150 Minuten, ≥ 180 Minuten, ≥ 210 Minuten und ≥ 240 Minuten bestehenden Gruppe ausgewählt ist.

8. Verfahren nach Anspruch 1, wobei ein oder mehrere der Pufferlösungen ein organisches Lösungsmittel, gegebenenfalls Glycerin, umfassen.

9. Verfahren nach Anspruch 1, wobei das chaotrope Salz aus der aus Natriumperchlorat, Lithiumchlorid, Magnesiumchlorid und Natriumchlorid bestehenden Gruppe ausgewählt ist.

10. Verfahren nach einem der Ansprüche 1 bis 9, wobei das Aptamer unter Verwendung eines Verfahrens detektiert und gegebenenfalls quantifiziert wird, das aus der aus Q-PCR, MS, "Next Generation"-Sequenzieren und -Hybridisieren bestehenden Gruppe ausgewählt ist, wobei die Q-PCR gegebenenfalls unter Verwendung von TaqMan®-PCR, eines interkalierenden fluoreszierenden Farbstoffs während des PCR-Verfahrens oder eines molekularen Leuchtsignals während des PCR-Verfahrens durchgeführt wird.

11. Verfahren nach einem der Ansprüche 1 bis 10, wobei das Aptamer eine detektierbare Gruppierung umfasst, wobei die detektierbare Gruppierung gegebenenfalls aus der aus einem Farbstoff, einem Quantenpunkt, einer Radiomarkierung, einer elektrochemischen funktionellen Gruppe und einem Enzym plus einem detektierbaren Enzymsubstrat bestehenden Gruppe ausgewählt ist, wobei der Farbstoff vorzugsweise ein fluoreszierender Farbstoff ist.

12. Verfahren nach einem der Ansprüche 1 bis 11, wobei das Aptamer eine einsträngige Nucleinsäure oder eine doppelsträngige Nucleinsäure ist.

13. Verfahren nach Anspruch 12, wobei das Aptamer DNA, RNA oder sowohl DNA als auch RNA umfasst.

14. Verfahren nach einem der Ansprüche 1 bis 13, wobei das Zielmolekül aus der aus einem Protein, einem Peptid, einem Kohlenhydrat, einem Polysaccharid, einem Glykoprotein, einem Hormon, einem Rezeptor, einem Antigen, einem Antikörper, einem Virus, einem Substrat, einem Metaboliten, einem Übergangszustandsanalogon, einem Cofaktor, einem Hemmer, einem Arzneimittel, einem Farbstoff, einem Nährstoff, einem Wachstumsfaktor, einem Gewebe und einem Betäubungsmittel bestehenden Gruppe ausgewählt ist, wobei das Zielmolekül vorzugsweise ein Protein oder ein Peptid ist.

15. Verfahren nach einem der Ansprüche 1 bis 14, wobei die Testprobe aus der aus einer biologischen Probe, einer Umweltprobe, einer chemischen Probe, einer pharmazeutischen Probe, einer Nahrungsmittelprobe, einer landwirtschaftlichen Probe und einer Veterinärprobe bestehenden Gruppe ausgewählt ist, wobei die biologische Probe gegebenenfalls aus der aus Blut, Vollblut, Leukozyten, mononukleären Zellen des peripheren Blutes, Plasma, Serum, Sputum, Atem, Urin, Sperma, Speichel, Meningealflüssigkeit, Fruchtwasser, Drüsenflüssigkeit, Lymphflüssigkeit, Brustwarzenaspirat, Bronchialaspirat, Synovialflüssigkeit, Gelenksaspirat, Zellen, einem zellulären Extrakt, Stuhl, Gewebe, einem Gewebeextrakt, einer Gewebebiopsie und Cerebrospinalflüssigkeit bestehenden Gruppe, vorzugsweise Plasma oder Serum, ausgewählt ist.

16. Verfahren nach einem der Ansprüche 1 bis 15, wobei die erste Markierung und die zweite Markierung, das erste Fangelement und das zweite Fangelement jeweils zumindest eine Komponente umfassen, die unabhängig aus der aus einem Polynucleotid, einem Polypeptid, einer Peptidnucleinsäure, einer Locked-Nucleinsäure, einem Oligosaccharid, einem Polysaccharid, einem Antikörper, einem Affibody, einem Antikörpermimetikum, einem Zellrezeptor, einem Liganden, einem Lipid, Biotin, Avidin, Streptavidin, Extravidin, Neutravidin, Traptavidin, einem Metall, Histidin und einem beliebigen Teil einer dieser Strukturen bestehenden Gruppe ausgewählt ist.

17. Verfahren nach Anspruch 1 oder 2, wobei die erste Markierung eine freisetzbare Gruppierung umfasst, wobei die freisetzbare Gruppierung vorzugsweise eine lichtspaltbare Gruppierung umfasst.

18. Verfahren nach Anspruch 1 oder 2, wobei der erste feste Träger und der zweite feste Träger jeweils unabhängig voneinander aus der aus einem Polymerkügelchen, einem Agarosekügelchen, einem Polystyrolkügelchen, einem Acrylamidkügelchen, einem Kügelchen mit festem Kern, einem porösen Kügelchen, einem paramagnetischen Kügelchen, einem Glaskügelchen, einem Kügelchen mit kontrollierten Poren, einem Mikrotiter-Well, einem Cycloolefin-Copolymersubstrat, einer Membran, einem Kunststoffsubstrat, Nylon, einer Langmuir-Blodgett-Schicht, Glas, einem Germaniumsubstrat, einem Siliciumsubstrat, einem Silicium-Waferchip, einem Durchfluss-Chip, einem Mikrokügelchen, einem Nanopartikel, einem Polytetrafluorethylensubstrat, einem Polystyrolsubstrat, einem Galliumarsenidsubstrat, einem Goldsubstrat und einem Silbersubstrat bestehenden Gruppe ausgewählt sind.

19. Verfahren nach Anspruch 1 oder 2, weiters umfassend das Quantifizieren des Ziels durch Quantifizieren des Aptamers.

20. Verfahren nach Anspruch 1 oder 2, wobei Detektion des Aptamers Hybridisierung des Aptamers an einen dritten festen Träger umfasst, wobei der dritte feste Träger eine Vielzahl von adressierbaren Merkmalen umfasst und wobei zumindest eines der Merkmale zumindest ein darauf angeordnetes Fangelement umfasst, das komplementär zu jeglicher innerhalb des Aptamers enthaltenen Sequenz ist, und/oder weiters den Schritt des Detektierens des Zielmoleküls durch Detektieren des Aptamerteils des Aptamer-Ziel-Affinitätskomplexes umfasst.

## Revendications

1. Procédé comprenant :
l'utilisation d'un aptamère qui est immobilisé sur un premier support solide, l'aptamère ayant une affinité de liaison spécifique pour la molécule cible et portant une première étiquette ayant une affinité pour un premier élément de capture, le premier support solide comprenant un premier élément de capture, et la première étiquette étant associée au premier élément de capture pour immobiliser l'aptamère sur le premier support solide,
ledit premier support solide ayant été lavé avec une ou plusieurs solutions qui dissocient les aptamères agrégés ;
la mise en contact dudit aptamère immobilisé avec l'échantillon à tester, un complexe d'affinité d'aptamère-cible étant formé si ladite molécule cible est présente dans ledit échantillon à tester ;
l'élimination d'un ou de plusieurs composants du mélange non associés audit premier support solide ;
l'attachement d'une seconde étiquette avec une affinité pour un second élément de capture à ladite molécule cible dans le complexe d'affinité d'aptamère-cible ;
la libération du complexe d'affinité d'aptamère-cible dudit premier support solide ;
l'exposition du complexe d'affinité d'aptamère-cible libéré à un deuxième support solide comprenant un second élément de capture et permettant d'associer la seconde étiquette audit second élément de capture ;
l'élimination de tous les composants du mélange non associés audit deuxième support solide ; et
l'élution des aptamères à partir dudit deuxième support solide avec une ou plusieurs solutions comprenant un sel chaotropique qui perturbe les interactions aptamère/analyte.

2. Procédé de détection de la présence de, ou de détermination de la quantité de, une molécule cible dans un échantillon, le procédé comprenant :
l'utilisation d'une pluralité d'aptamères immobilisés, chacun desdits aptamères étant spécifique à une molécule cible, et chacun de ladite pluralité d'aptamères comprenant une première étiquette de capture clivable, lesdits aptamères étant immobilisés sur un support solide ayant des sondes qui adhèrent à sa surface, les sondes se liant à la première étiquette, de sorte que les aptamères sont immobilisés sur le support solide par la liaison de la première étiquette et de la sonde ;
la mise en contact des aptamères immobilisés avec un échantillon contenant des molécules cibles pour former un mélange contenant des complexes d'aptamère-molécule cible liés au support solide ;
la séparation des complexes d'aptamère-molécule cible liés au support solide du reste du mélange ;
l'introduction d'une seconde étiquette de capture sur le composant molécule cible des complexes d'aptamère-molécule cible ;
la dissociation des complexes d'aptamère-molécule cible de la surface du support solide par clivage de la première étiquette de capture clivable ;
l'utilisation d'un deuxième support solide ayant des sondes qui adhèrent à la surface du support, les sondes étant capables de se lier à la seconde étiquette de capture sur les molécules cibles ;
la mise en contact des complexes d'aptamère-molécule cible dissociés avec le deuxième support solide de sorte que les complexes d'aptamère-molécule cible se lient au second support par liaison de la seconde étiquette de capture et de la sonde ;
l'élution des aptamères à partir dudit deuxième support solide avec une ou plusieurs solutions tamponnées comprenant un sel chaotropique qui perturbe les interactions aptamère/analyte mais supporte les interactions aptamère/aptamère et l'hybridation d'ADN ;
la dissociation de l'aptamère-molécule cible ;
la détection des aptamères libres.

3. Procédé selon la revendication 1 ou la revendication 2, dans lequel ledit aptamère comprend au moins un nucléotide modifié en C-5.

4. Procédé selon la revendication 1, 2 ou 3, dans lequel ledit aptamère comprend au moins une modification chimique comprenant une substitution chimique en une ou plusieurs positions indépendamment choisies parmi une position ribose, une position désoxyribose, une position phosphate et une position de base, dans lequel facultativement ladite modification chimique est indépendamment choisie dans le groupe constitué par une modification par un sucre en position 2', une modification 2'-amino (2'-NH₂), une modification 2'-fluoro (2'-F), une modification 2'-0-méthyle (2'-OMe), une modification par une pyrimidine en position 5 et une modification par une purine en position 8, une modification sur une amine exocyclique cytosine, une substitution de 5-bromouracil, une substitution de 5-bromodésoxyuridine, une substitution de 5-bromodésoxycytidine, une modification du squelette, une méthylation, une coiffe en 3' et une coiffe en 5'.

5. Procédé selon l'une quelconque des revendications 1 à 4, comprenant en outre l'introduction d'un challenge cinétique.

6. Procédé selon l'une quelconque des revendications 1 à 5, dans lequel ledit complexe d'affinité d'aptamère-cible a un taux de dissociation faible.

7. Procédé selon la revendication 6, dans lequel le taux de dissociation dudit complexe d'affinité d'aptamère-cible (t_{1/2}) est :
(a) supérieur ou égal à 30 minutes ;
(b) compris entre environ 30 minutes et environ 240 minutes ; ou
(c) choisi dans le groupe constitué par ≥ 30 minutes, ≥ 60 minutes, ≥ 90 minutes, ≥ 120 minutes, ≥ 150 minutes, ≥ 180 minutes, ≥ 210 minutes et ≥ 240 minutes.

8. Procédé selon la revendication 1, dans lequel une ou plusieurs desdites solutions tamponnées comprennent un solvant organique, facultativement du glycérol.

9. Procédé selon la revendication 1, dans lequel ledit sel chaotropique est choisi dans le groupe constitué par le perchlorate de sodium, le chlorure de lithium, le chlorure de magnésium et le chlorure de sodium.

10. Procédé selon l'une quelconque des revendications 1 à 9, dans lequel ledit aptamère est détecté et facultativement quantifié en utilisant un procédé choisi dans le groupe constitué par la Q-PCR, la SM, le séquençage de nouvelle génération et l'hybridation, ladite Q-PCR étant facultativement réalisée en utilisant une PCR TaqMan®, un colorant fluorescent intercalant pendant le processus de PCR, ou une balise moléculaire pendant le processus de PCR.

11. Procédé selon l'une quelconque des revendications 1 à 10, dans lequel ledit aptamère comprend une fraction détectable, ladite fraction détectable étant facultativement choisie dans le groupe constitué par un colorant, un point quantique, un radiomarqueur, un groupe fonctionnel électrochimique et une enzyme plus un substrat enzymatique détectable, ledit colorant étant de préférence un colorant fluorescent.

12. Procédé selon l'une quelconque des revendications 1 à 11, dans lequel ledit aptamère est un acide nucléique monobrin ou un acide nucléique double brin.

13. Procédé selon la revendication 12, dans lequel ledit aptamère comprend de l'ADN, de l'ARN ou à la fois de l'ADN et de l'ARN.

14. Procédé selon l'une quelconque des revendications 1 à 13, dans lequel ladite molécule cible est choisie dans le groupe constitué par une protéine, un peptide, un glucide, un polysaccharide, une glycoprotéine, une hormone, un récepteur, un antigène, un anticorps, un virus, un substrat, un métabolite, un analogue de l'état de transition, un cofacteur, un inhibiteur, un médicament, un colorant, un nutriment, un facteur de croissance, un tissu et une substance réglementée, de préférence dans lequel ladite molécule cible est une protéine ou un peptide.

15. Procédé selon l'une quelconque des revendications 1 à 14, dans lequel ledit échantillon à tester est choisi dans le groupe constitué par un échantillon biologique, un échantillon environnemental, un échantillon chimique, un échantillon pharmaceutique, un échantillon alimentaire, un échantillon agricole et un échantillon vétérinaire, dans lequel facultativement l'échantillon biologique est choisi dans le groupe constitué par le sang, le sang total, les leucocytes, les cellules mononucléaires de sang périphérique, le plasma, le sérum, les expectorations, l'haleine, l'urine, le sperme, la salive, le liquide méningé, le liquide amniotique, le liquide glandulaire, le liquide lymphatique, le liquide d'aspiration du mamelon, le liquide d'aspiration bronchique, le liquide synovial, le liquide d'aspiration articulaire, les cellules, un extrait cellulaire, les selles, un tissu, un extrait tissulaire, une biopsie tissulaire et le liquide cérébrospinal, de préférence le plasma ou le sérum.

16. Procédé selon l'une quelconque des revendications 1 à 15, dans lequel ladite première étiquette et ladite seconde étiquette, ledit premier élément de capture et ledit second élément de capture comprennent chacun au moins un composant indépendamment choisi dans le groupe constitué par un polynucléotide, un polypeptide, un acide nucléique peptidique, un acide nucléique bloqué, un oligosaccharide, un polysaccharide, un anticorps, un afficorps, un mimétique d'anticorps, un récepteur cellulaire, un ligand, un lipide, la biotine, l'avidine, la streptavidine, l'extravidine, la neutravidine, la traptavidine, un métal, l'histidine, et n'importe quelle partie de n'importe laquelle de ces structures.

17. Procédé selon la revendication 1 ou la revendication 2, dans lequel la première étiquette comprend une fraction libérable, de préférence dans lequel la fraction libérable comprend une fraction photoclivable.

18. Procédé selon la revendication 1 ou la revendication 2, dans lequel ledit premier support solide et ledit deuxième support solide sont chacun indépendamment choisis dans le groupe constitué par une bille de polymère, une bille d'agarose, une bille de polystyrène, une bille d'acrylamide, une bille de noyau solide, une bille poreuse, une bille paramagnétique, une bille de verre, une bille de pore contrôlée, un puits de microtitrage, un substrat de copolymère cyclooléfinique, une membrane, un substrat plastique, un nylon, un film de Langmuir-Blodgett, un verre, un substrat de germanium, un substrat de silicium, une puce de plaquette de silicium, une puce à flux traversant, une microbille, une nanoparticule, un substrat de polytétrafluoroéthylène, un substrat de polystyrène, un substrat de gallium-arséniure, un substrat d'or et un substrat d'argent.

19. Procédé selon la revendication 1 ou la revendication 2, comprenant en outre la quantification de ladite cible par quantification dudit aptamère.

20. Procédé selon la revendication 1 ou la revendication 2, dans lequel la détection de l'aptamère comprend l'hybridation de l'aptamère sur un troisième support solide, le troisième support solide comprenant une pluralité de caractéristiques adressables et au moins une desdites caractéristiques comprenant au moins un élément de capture disposé sur celui-ci qui est complémentaire d'une quelconque séquence contenue dans l'aptamère et/ou comprenant en outre l'étape de détection de ladite molécule cible par détection de la partie aptamère dudit complexe d'affinité d'aptamère-cible.
